# EUROPEAN PATENT APPLICATION

(11) **EP 4 458 969 A1**
(43) Date of publication of application: **06.11.2024**
(21) Application number: 22915072.7
(22) Date of filing: 29.12.2022
(51) Int. Cl.: C12N 15/113, C12N 15/86, C12N 5/10, A61K 48/00, A61K 31/713, A61P 31/18

(54) **SIRNA AND SHRNA TARGETING HIV, AND CORRESPONDING COMBINATION, EXPRESSION CASSETTE, CELL, AND APPLICATION THEREOF**

(30) Priority: 31.12.2021 CN 202111666495
(71) Applicant: Beijing SoloBio Genetechnology Company Ltd., Beijing 100176 (CN)
(72) Inventor: LI, Cui, Beijing 100176 (CN); ZHANG, Yue, Beijing 100176 (CN); FAN, Jundie, Beijing 100176 (CN)
(74) Representative: Casalonga
(86) International application number: PCT/CN2022/143442
(87) International publication number: WO 2023/125823

(57) **Abstract**

Provided is an RNA, siRNA, shRNA or nucleic acid coding for shRNA, combination of RNAs, combination of siRNAs, combination of shRNAs, or combination of the nucleic acids coding for shRNAs targeting HIV Also provided are an expression cassette, recombinant vector, recombinant virus particle, kit, cell or pharmaceutical composition comprising the above sequences or combinations and their use for preparing drugs for diagnosis, treatment and/or prevention of HIV infection or AIDS.

## Description

### TECHNICAL FIELD

The invention relates to the field of biomedicine, more particularly, to RNA or combination thereof, siRNA or combination thereof, shRNA or combination thereof, nucleic acid coding for shRNA or combination thereof, expression cassette, vector, cell, pharmaceutical composition targeting HIV and use thereof.

### BACKGROUND

AIDS caused by HIV (Human Immunodeficiency virus) infection has spread around the world at an unusually rapid speed since it was first discovered in the United States in 1981. HIV, which belongs to retroviridae, is an RNA retrovirus. HIV is an enveloped virus consisting of two single-stranded positive-stranded RNA molecules (+ssRNA). After invading cells, the viral RNA of HIV is reverse transcribed into single-stranded cDNA under the action of its own reverse transcriptase, which is then formed into a double-stranded cDNA in host cell. The double-stranded cDNA is then integrated into the host cell chromosomes, and pathogenic mRNA/gRNA is generated by using host cell transcription and synthesis systems. The main target of HIV is CD4 positive cells, such as CD4+T cells. In the world, nearly 20 kinds of anti-AIDS drugs have been approved for clinical use until present. However, none of the present drugs can completely remove HIV virus in human body. Although the "cocktail therapy" in which multiple drugs are used in combination exhibits better effect than single drug treatment, however, the virus still cannot be completely removed. After drug treatment, probably HIV can be reactivated and amplified in CD4 + cells carrying HIV genome.

RNA interference (RNAi), which is a mechanism for inhibiting gene expression, is a specific RNA degradation process induced by double-stranded short interfering RNA (siRNA). It is a process occurring after gene transcription, that is, regulating gene expression at mRNA level, which can efficiently and specifically block the expression of target genes. Small interfering RNA (small interfering RNA; siRNA), also called short interfering RNA (short interfering RNA) or silencing RNA (silencing RNA), is a double-stranded RNA with the length of 18 to 25 nucleotides, which is designed based on target genes and synthesized by chemical methods, for the purpose of transiently transfecting cells. Alternatively, the siRNA for the target gene can also be designed as a hairpin structure and sent into the body, and then the hairpin structure is expressed to form shRNA. shRNA is the abbreviation of short hairpin RNA, translated as "short hairpin RNA". shRNA comprises two short reverse repeats sequences. At present, it is known that shRNA is mainly involved in RNA interference (RNAi) and regulates gene expression in a specific manner. After entering cells, shRNA is unwinded into sense RNA strand and antisense RNA strand by RNA helicase in host cells. Antisense RNA strand is incorporated with some enzymes in vivo to form silencing complex RISC, which can recognize and bind to mRNA comprising the complementary sequence thereof. After binding, RISC could function as a nuclease, cleave and degrade the mRNA, thereby inhibit the expression of the target gene.

So far, researchers have designed siRNAs which target HIV-1 coding sequence and non-coding sequence(nef, tat, gag, vif, env, rev, LTR) (Boden et al., 2004; Capodici et al., 2002; Novina et al., 2002; Han et al., 2004; Jacque et al., 2002; Lee et al., 2002; Novina et al., 2002). However, the present interference method is only effective in short term. Due to high mutation rate of HIV genome and high specificity of siRNA, these siRNAs will lose their functions easily, which makes it difficult for RNAi to inhibit HIV replication for long time (Das et al., 2004; Westerhout et al., 2005).

Therefore, there is a need in the art for a drug and a method capable of inhibiting HIV escape mutants for long time, so as to solve the problems existing in the prior art.

### SUMMARY

Therefore, the invention provides an RNA, siRNA, shRNA or nucleic acid coding for shRNA targeting HIV, which can significantly and effectively inhibit the replication of HIV virus strains and drug-resistant mutant virus strains. In particular, the invention also provides a combination of RNAs, combination of siRNAs, combination of shRNAs, or combination of the nucleic acids coding for shRNAs, which can not only efficiently inhibit the replication of HIV virus strains or drug-resistant mutant virus strains, but also further effectively inhibit the generation of HIV escape mutant strains for long time, and has a good therapeutic effect on HIV positive patients. Exemplary RNA, siRNA, shRNA and the nucleic acid coding therefor involved in the invention are shown in Tables 1 and 2.

In one aspect, the present invention provides an RNA targeting HIV, which targets a sequence shown in any one of SEQ ID NOs: 1-5. In another aspect, the present invention provides an RNA, which sequence is shown in any one of SEQ ID NOs: 6-10 or 20-24.

In one aspect, the present invention provides a siRNA targeting HIV, the siRNA comprising a sense strand and an antisense strand that is reverse complementary to the sense strand, consisting of the following sequences:
(i) the sequence of the sense strand shown in SEQ ID NO: 6, and the sequence of the antisense strand shown in SEQ ID NO: 20;
(ii) the sequence of the sense strand shown in SEQ ID NO: 7, and the sequence of the antisense strand shown in SEQ ID NO: 21;
(iii) the sequence of the sense strand shown in SEQ ID NO: 8, and the sequence of the antisense strand shown in SEQ ID NO: 22;
(iv) the sequence of the sense strand shown in SEQ ID NO: 9, and the sequence of the antisense strand shown in SEQ ID NO: 23; or
(v) the sequence of the sense strand shown in SEQ ID NO: 10, and the sequence of the antisense strand shown in SEQ ID NO: 24.

In another aspect, the present invention provides an shRNA targeting HIV or a nucleic acid coding for the shRNA, the sequence of the shRNA comprises:
(i) the sequence of SEQ ID NO: 6 and SEQ ID NO: 20;
(ii) the sequence of SEQ ID NO: 7 and SEQ ID NO: 21;
(iii) the sequence of SEQ ID NO: 8 and SEQ ID NO: 22;
(iv) the sequence of SEQ ID NO: 9 and SEQ ID NO: 23; or
(v) the sequence of SEQ ID NO: 10 and SEQ ID NO: 24.

In a preferred embodiment, the sequence of the shRNA is shown in any one of SEQ ID NOs: 25-29.

In a preferred embodiment, the nucleic acid coding for the shRNA is shown in any one of SEQ ID NOs: 11-15.

In another aspect, the present invention also provides a combination of RNAs as described above. In a preferred embodiment, the combination of RNAs comprises:
(i) an RNA targeting the Gag gene and an RNA targeting the LTR gene, wherein the sequence of RNA targeting the Gag
   gene is shown in SEQ ID NO: 6 or 20, the sequence of RNA targeting the LTR gene is shown in SEQ ID: 9 or 23;
(ii) an RNA targeting the Gag gene and an RNA targeting the LTR gene, wherein the sequence of RNA targeting the Gag
   gene is shown in SEQ ID NO: 7 or 21, the sequence of RNA targeting the LTR gene is shown in SEQ ID: 9 or 23;
(iii) an RNA targeting the Gag gene and an RNA targeting the LTR gene, wherein the sequence of RNA targeting the Gag
   gene is shown in SEQ ID NO: 8 or 22, the sequence of RNA targeting the LTR gene is shown in SEQ ID: 9 or 23; or
(iv) an RNA targeting the Gag gene and an RNA targeting the Nef gene, wherein the sequence of RNA targeting the Gag
   gene is shown in SEQ ID NO: 7 or 21, the sequence of RNA targeting the Nef gene is shown in SEQ ID: 10 or 24.

In another aspect, the present invention also provides a combination of siRNAs as described above. In a preferred embodiment, the combination of siRNAs comprises:
(i) a siRNA targeting the Gag gene and a siRNA targeting the LTR gene, wherein the sense strand sequence of the siRNA
   targeting the Gag gene is shown in SEQ ID NO: 6, the antisense strand sequence is shown in SEQ ID NO: 20, and the sense strand sequence of the siRNA targeting the LTR gene is shown in SEQ ID NO: 9, the antisense strand sequence is shown in SEQ ID NO: 23;
(ii) a siRNA targeting the Gag gene and a siRNA targeting the LTR gene, wherein the sense strand sequence of the siRNA
   targeting the Gag gene is shown in SEQ ID NO: 7, the antisense strand sequence is shown in SEQ ID NO: 21, and the sense strand sequence of the siRNA targeting the LTR gene is shown in SEQ ID NO: 9, the antisense strand sequence is shown in SEQ ID NO: 23;
(iii) a siRNA targeting the Gag gene and a siRNA targeting the LTR gene, wherein the sense strand sequence of the siRNA
   targeting the Gag gene is shown in SEQ ID NO: 8, the antisense strand sequence is shown in SEQ ID NO: 22, and the sense strand sequence of the siRNA targeting the LTR gene is shown in SEQ ID NO: 9, the antisense strand sequence is shown in SEQ ID NO: 23; or
(iv) a siRNA targeting the Gag gene and a siRNA targeting the Nef gene, wherein the sense strand sequence of the
   siRNA targeting the Gag gene is shown in SEQ ID NO: 7, the antisense strand sequence is shown in SEQ ID NO: 21, and the sense strand sequence of the siRNA targeting the Nef gene is shown in SEQ ID NO: 10, the antisense strand sequence is shown in SEQ ID NO: 24.

In another aspect, the present invention also provides a combination of shRNAs targeting HIV In a preferred embodiment, the combination of shRNAs comprises:
(i) a shRNA targeting the Gag gene and a shRNA targeting the LTR gene, wherein the sequence of the shRNA targeting
   The Gag gene is shown in SEQ ID NO: 25 and the sequence of the shRNA targeting the LTR gene is shown in SEQ ID NO: 28;
(ii) a shRNA targeting the Gag gene and a shRNA targeting the the LTR gene, wherein the sequence of the shRNA
   targeting the Gag gene is shown in SEQ ID NO: 26 and the sequence of the shRNA targeting the LTR gene is shown in SEQ ID NO: 28;
(iii) a shRNA targeting the Gag gene and a shRNA targeting the LTR gene, wherein the sequence of the shRNA targeting
   The Gag gene is shown in SEQ ID NO: 27 and the sequence of the shRNA targeting the LTR gene is shown in SEQ ID NO: 28; or
(iv) a shRNA targeting the Gag gene and a shRNA targeting the Nef gene, wherein the sequence of the shRNA targeting
   the Gag gene is shown in SEQ ID NO: 26 and the sequence of the shRNA targeting the Nef gene is shown in SEQ ID NO: 29.

In another aspect, the present invention also provides a combination of the nucleic acids coding for shRNAs as described above. In a preferred embodiment, the combination of the nucleic acids coding for shRNAs comprises:
(i) a nucleic acid coding for shRNA targeting the Gag gene and a nucleic acid coding for shRNA targeting the LTR gene,
   wherein the sequence of the nucleic acid coding for shRNA targeting the Gag gene is shown in SEQ ID NO: 11 and the sequence of the nucleic acid coding for shRNA targeting the LTR gene is shown in SEQ ID NO: 14,
(ii) a nucleic acid coding for shRNA targeting the Gag gene and a nucleic acid coding for shRNA targeting the LTR gene,
   wherein the sequence of the nucleic acid coding for shRNA targeting the Gag gene is shown in SEQ ID NO: 12 and the sequence of the nucleic acid coding for shRNA targeting the LTR gene is shown in SEQ ID NO: 14,
(iii) a nucleic acid coding for shRNA targeting the Gag gene and a nucleic acid coding for shRNA targeting the LTR gene,
   wherein the sequence of the nucleic acid coding for shRNA targeting the Gag gene is shown in SEQ ID NO: 13 and the sequence of the nucleic acid coding for shRNA targeting the LTR gene is shown in SEQ ID NO: 14, or
(iv) a nucleic acid coding for shRNA targeting the Gag gene and a nucleic acid coding for shRNA targeting the Nef gene,
wherein the sequence of the nucleic acid coding for shRNA targeting the Gag gene is shown in SEQ ID NO: 12 and the sequence of the nucleic acid coding for shRNA targeting the Nef gene is shown in SEQ ID NO: 15.

In another aspect, the invention also provides an expression cassette containing the above-mentioned nucleic acids. In another aspect, the invention also provides a vector comprising the nucleic acids, or the expression cassette above. In another aspect, the invention also provides a cell comprising the aforementioned RNA or combination thereof, siRNA or combination thereof, shRNA or combination thereof, nucleic acid coding for shRNA or combination thereof, expression cassette or vector. In another aspect, the present invention also provides a pharmaceutical composition comprising the aforementioned RNA or combination thereof, siRNA or combination thereof, shRNA or combination thereof, nucleic acid coding for shRNA or combination thereof, expression cassette, vector or cell. In another aspect, the sequence of the nucleic acid coding for shRNA of the present invention is DNA sequence.

### DEFINITION

Unless otherwise indicated, the practice of the present invention will use conventional chemical, biochemical, recombinant DNA technologies and immunological methods in the art. Such technologies are well interpreted in the literature (see, for example, Fundamental Virology, second edition, vol. I & II (edited by B.N. Fields and D.M. Knipe); Handbook of Experimental Immunology, Vols. I-FV (edited by D.M. Weir and CC. Blackwell, Blackwell Scientific Publications); T.E. Creighton, Proteins: Structures and Molecular Properties (W.H. Freeman and Company, 1993); A.L. Lehninger, Biochemistry (Worth Publishers, Inc., current addition); Sambrook et al., Molecular Cloning: A Laboratory Manual (second edition, 1989); Methods In Enzymology (edited by S. Colowick and N. Kaplan, Academic Press, Inc.).

In order to facilitate the understanding of various embodiments of the present disclosure, following explanations for specific terms are provided.

Synthesis: Produced by artificial means in laboratory. For example, synthetic nucleic acids can be chemically synthesized in the laboratory.

Isolated: "Isolated" biological components (e.g. nucleic acid molecules, proteins, viruses, or cells) have been substantially isolated or purified from other biological components (e.g. other chromosomal and extrachromosomal DNA and RNA, proteins and cells) in the organism and the cells or tissues thereof in which the components are naturally present. Nucleic acid molecules and proteins that have been "isolated" include those purified by standard purification methods. The term also includes nucleic acid molecules and proteins prepared through recombinant expression in host cells and by chemically synthesized.

Recombinant: A recombinant nucleic acid molecule is a nucleic acid molecule that has a sequence not naturally occurring or generated by artificial combination of two fragments (which are otherwise separated with each other). The artificial combination can be achieved by chemical synthesis or by artificial manipulation of isolated nucleic acid molecular fragments (such as through genetic engineering technology).

Likewise, a recombinant virus is a virus comprising a sequence that is not naturally occurring or that is generated by artificial combination of at least two sequences from different origins. The term "recombinant" also includes nucleic acids, proteins, and viruses that have been modified by the addition, substitution, or deletion of a portion on a naturally occurring nucleic acid molecule, protein, or virus. "Recombinant SIV" as used herein refers to an SIV particle having a recombinant nucleic acid molecule (e.g. a recombinant nucleic acid molecule coding for shRNA) packaged therein.

Sequence identity: Identity or similarity between two or more nucleic acid or between two or more polypeptides is represented according to the identity or similarity between their sequences. Sequence identity can be measured as percentage identity. The higher the percentage, the higher the identity between the sequences. Sequence similarity can be measured as percentage similarity (conserved amino acid substitutions are taken into consideration), the higher the percentage, the higher the similarity between the sequence. Homologues or orthologous homologues of nucleic acid or amino acid sequences have a relatively high sequence identity/similarity when aligned using standard methods. The homology between orthologous proteins or cDNAs derived from species that are more closely related (e.g. human and mouse sequences) is much more higher than that from species that are less related (e.g. human and nematode (C. elegans) sequences).

The scale of sequence identity comparison may be over the full-length of the genome, the full-length of a gene coding sequence, or a fragment of at least about 500 to 5000 nucleotides, is desired. However, identity among smaller fragments (e.g. of at least about 9 nucleotides, usually at least about 20 to 24 nucleotides, at least about 28 to 32 nucleotides, at least about 36 or more nucleotides) may also be desired. Generally, when referring to "identity", "homology" or "similarity" between two different sequences, "identity", "homology" or "similarity" is determined in reference to "aligned" sequences. "aligned" sequences or "alignments" refers to multiple nucleic acid sequences or protein (amino acids) sequences which often contain corrections for missing or additional bases or amino acids as compared to a reference sequence.

Alignments are performed by using any of a variety of publicly or commercially available Multiple Sequence Alignment Programs. Sequence alignment programs are available for amino acid sequences alignment, e.g., the "Clustal X", "MAP", "PIMA", "MSA", "BLOCKMAKER", "MEME" and "Match-Box" programs. Generally, any of these programs are used at default settings although one of skilled in the art can alter these settings as needed. Alternatively, one of skilled in the art can utilize another algorithm or computer program which provides at least the level of identity or alignment as that provided by the referenced algorithms and programs. See, e.g., J. D. Thomson et al., Nucl. Acids. Res., "Acomprehensive comparison of multiple sequence alignments", 27 (13): 2682-2690 (1999).

Multiple sequence alignment programs are also available for nucleic acid sequences alignment. Examples of such programs include "Clustal W," "CAPSequence Assembly," "BLAST," "MAP," and "MEME," which are accessible through Web servers on the Internet. Other sources for such programs are known to those of skilled in the art. Alternatively, the Vector NTI utilities are also used. There are also a number of algorithms known in the art that can be used to measure nucleotide sequence identity, including those contained in the programs described above. As another example, polynucleotide sequences can be compared using Fasta ^{™} (a program in GCG Version 6.1). Fasta ^{™} provides alignments and percent sequence identity of the regions of the best overlap between the query and search sequences. For instance, percentage sequence identity between nucleic acid sequences can be determined using Fasta ^{™} with default parameters (a word size of 6, and the NOPAM factor for the scoring matrix) as provided in GCG Version 6.1 (herein incorporated by reference).

Operably linked: A first nucleic acid sequence is operably linked with a second nucleic acid sequence when the first nucleic acid sequence is placed in a functional relationship with the second nucleic acid sequence. For example, a promoter is operably linked with a coding sequence if the promoter affects the transcription or expression of the coding sequence. Generally, operably linked DNA sequences are contiguous, and where necessary to join two protein coding regions, in the same reading frame.

Vector: A vector is a nucleic acid molecule that allows insertion of an exogenous nucleic acid without disrupting the ability of the vector to replicate and/or integrate in host cell. A vector may contain nucleic acid sequences that allow it to replicate in a host cell, such as a replication origin. The vector may also contain one or more selective marker genes and other genetic elements. Expression vectors are vectors that contain the necessary regulatory sequences to allow transcription and translation of the inserted gene. In some embodiments, the vector is an SIV vector.

A "Replication-defective virus" or "viral vector" refers to a synthetic or artificial viral particle in which an expression cassette containing a gene of interest is packaged in a viral capsid or envelope, where any viral genomic sequences also packaged within the virus capsid or envelope are replication-deficient; i.e., they cannot generate progeny virions, but retain the ability to infect target cells. In one embodiment, the genome of the viral vector does not include genes encoding the enzymes required for replication (the genome can be engineered to be "gutless"--containing only the transgene of interest flanked by the signals required for amplification and packaging of artificial genome), but these genes may be supplied during production. Therefore, it is deemed safe for use in gene therapy since replication and infection by progeny virions cannot occur except in the presence of the viral enzymes required for replication. Such replication-defective viruses may be adeno-associated viruses (AAV), adenoviruses, lentiviruses (integrating or non-integrating) or other suitable virus sources.

"Lentivirus" belongs to subgenus of retrovirus, represented by human immunodeficiency virus (HIV), which can infect dividing cells and integrate into their genomes, and also has the ability to infect neurons, macrophages, hepatocytes, cardiomyocytes and stem cells. " Lentiviral vector" is a viral vector derived from primate immunodeficiency virus, which has been widely used in gene therapy as an effective tool for gene transfer. Lentiviral vectors carrying exogenous genes achieve the expression of exogenous genes in cells or living tissues by infecting cells or living tissues. Examples of lentiviral vectors include recombinant lentiviral vectors of primates, such as human immunodeficiency virus (HIV) or simian immunodeficiency virus (SIV), or non-primate recombinant lentiviral vectors. "Simian immunodeficiency virus (SIV) vector" refers to a vector in which, among the nucleic acid molecules in the viral particle, sequences necessary to function as a viral vector are based on the SIV genome, and the necessary sequences refer to the sequences of, in order from the 5 'side, the R region and U5 region in the 5 'LTR, packaging signal (φ), RRE, and U3 region except the promoter region, and R region in the 3' LTR.

Promoter: DNA region that directs/causes transcription of nucleic acids (e.g. genes). The Promoter includes essential nucleic acid sequence near the transcription start site. Usually, the promoter is located near the gene to be transcribed. The promoter region also optionally comprises a distal enhancer or repressor elements which may be located thousands of base pairs away from the transcription start site.

Pharmaceutically acceptable carriers: The pharmaceutically acceptable carriers (vehicle) that can be used in this disclosure are conventional. Compositions and formulations suitable for drug delivery of one or more therapeutic compounds, molecules or reagents have been described in Remington's Pharmaceutical Sciences, by E.W. Martin, MackPublishing Co., Easton, PA, 15th Edition (1975).

Prevention, treatment, or remission of disease: "Prevention" of disease (e.g. HIV infection) means suppressing the full onset of the disease. "Treatment" means a therapeutic intervention that improves the signs or symptoms of a disease or pathological condition after the onset of the disease. "Remission" means reducing the number or severity of signs or symptoms of the disease.

Administration/provision: To provide or administer an agent, such as a therapeutic agent (e.g. recombinant SIV), to a subject by an effective route. Exemplary routes of administration include, but are not limited to, injection (e.g., subcutaneous, intramuscular, intradermal, intraperitoneal, and intravenous), oral, intra-catheter, sublingual, rectal, percutaneous, intranasal, vaginal, and inhalation routes.

Therapeutically effective amount: An amount of a specific drug or therapeutic agent (such as recombinant SIV) sufficient to achieve the desired effect in the subject or cell treated with the agent. The effective amount of the agent depends on a variety of factors including but not limited to the subject or cell being treated and the way of administration of the therapeutic compositions.

Subjects: Living multicellular vertebrate organisms, including human and non-human mammalian categories.

### RNA targeting HIV

The first aspect of the invention provides an RNA targeting HIV, the RNA targeting sequence is shown in any one of SEQ ID NOs: 1-5.

In a specific embodiment of the invention, the sequence of RNA is shown in any one of SEQ ID NOs: 6-10 or 20-24.

### siRNA targeting HIV

The second aspect of the invention provides a siRNA targeting HIV, comprising a sense strand and an antisense strand inversely complementary thereto, wherein the siRNA targets the Gag gene, the LTR gene and/or the Nef gene of HIV

In a specific embodiment of the invention, the siRNA targeting sequence is shown in any one of SEQ ID NOs: 1-5.

In one embodiment of the present invention, the siRNA sense strand sequence targeting the Gag gene of HIV is selected from the sequences consisting of 19-23 continuous nucleotides in any one of SEQ ID NOs: 1-3; The siRNA sense strand sequence targeting the LTR gene of HIV is selected from the sequences consisting of 19-26 continuous nucleotides in SEQ ID NO: 4; The siRNA sense strand sequence targeting the Nef gene of HIV is selected from the sequences consisting of 19-25 continuous nucleotides in SEQ ID NO: 5. In another embodiment of the invention, the antisense strand of the siRNA is a sequence that is reversely complementary to the sense strand.

In one embodiment of the invention, the siRNA targeting the Gag gene consists of the following sequences:
(i) the sequence of the sense strand is shown in SEQ ID NO: 6, and the sequence of the antisense strand is shown in SEQ ID NO: 20;
(ii) the sequence of the sense strand is shown in SEQ ID NO: 7, and the sequence of the antisense strand is shown in SEQ ID NO: 21; or
(iii) the sequence of the sense strand is shown in SEQ ID NO: 8, and the sequence of the antisense strand is shown in SEQ ID NO: 22.

In another embodiment of the invention, the sense strand sequence of the siRNA targeting the LTR gene is shown in SEQ ID
NO: 9, and the antisense strand sequence is shown in SEQ ID NO: 23.

In another embodiment of the invention, the sense strand sequence of the siRNA targeting the Nef gene is shown in SEQ ID
NO: 10, and the antisense strand sequence is shown in SEQ ID NO: 24.

### shRNA targeting HIV and nucleic acid coding therefor

The third aspect of the invention provides a shRNA and a nucleic acid coding for the shRNA, wherein the shRNA targeting the Gag gene, the LTR gene and/or the Nef gene of HIV.

In one embodiment of the invention, the shRNA is formed by linking the sense strand sequence of the siRNA as previously described and the antisense strand sequence inversely complementary thereto, through an intermediate non-complementary linker sequence, wherein the sense strand sequence and the antisense strand sequence form the stem region in a stem-loop structure and the intermediate non-complementary linker sequence forms the loop region in the stem-loop structure.

In one embodiment of the invention, the shRNA is processed *in vivo* to produce the siRNA as previously described.

In one embodiment of the invention, the sequence of the shRNA targeting the Gag gene comprises:
(i) the sequence shown in SEQ ID NO: 6 and SEQ ID NO: 20;
(ii) the sequence shown in SEQ ID NO: 7 and SEQ ID NO: 21; or
(iii) the sequence shown in SEQ ID NO: 8 and SEQ ID NO: 22.

In another embodiment of the invention, the sequence of said shRNA targeting the LTR gene comprises the sequence shown in SEQ ID NO: 9 and SEQ ID NO: 23.

In another embodiment of the invention, the sequence of said shRNA targeting the Nef gene comprises the sequence shown in SEQ ID NO: 10 and SEQ ID NO: 24.

In a preferred embodiment of the invention, the sequence of said shRNA is shown in any one of SEQ ID NOs: 25-29; the sequence of the nucleic acid coding for the shRNA is shown in any one of SEQ ID NOs: 11-15.

### Combination of RNAs targeting HIV

The fourth aspect of the invention also provides a combination of RNAs targeting HIV In one embodiment of the invention, the combination comprises at least one of the RNAs as previously described.

In another embodiment of the invention, the combination comprises at least two of the followings: the RNA targeting the Gag gene, the RNA targeting the LTR gene, or the RNA targeting the Nef gene.

In a preferred embodiment of the invention, the combination of RNAs targeting HIV comprises:
(i) an RNA targeting the Gag gene and an RNA targeting the LTR gene,
(ii) an RNA targeting the Gag gene and an RNA targeting the Nef gene, or
(iii) an RNA targeting the LTR gene and an RNA targeting the Nef gene.

Further preferably, the sequence of RNA targeting the Gag gene of HIV is selected from the sequences consisting of 19-23 continuous nucleotides in any one of SEQ ID NOs: 1-3 or the sequences inversely complementary thereto, more preferably, the sequence shown in any one of SEQ ID NOs: 6-8 or 20-22. The sequence of RNA targeting the LTR gene of HIV is selected from the sequences consisting of continuous 19-26 nucleotide sequence in SEQ ID NO: 4 or sequences inversely complementary thereto, more preferably, the sequence shown in SEQ ID NO: 9 or 23. The sequence of RNA targeting the Nef gene of HIV is selected from the sequences consisting of continuous 19-25 nucleotide sequence in SEQ ID NO: 5 or sequences inversely complementary thereto, more preferably, the sequence shown in SEQ ID NO: 10 or 24.

In a preferred embodiment of the invention, the RNAs targeting HIV comprises:
(i) an RNA targeting the Gag gene and an RNA targeting the LTR gene, wherein the sequence of RNA targeting the
   Gag gene is shown in SEQ ID NO: 6 or 20 and the sequence of RNA targeting the LTR gene is shown in SEQ ID NO: 9 or 23;
(ii) an RNA targeting the Gag gene and an RNA targeting the LTR gene, wherein the sequence of RNA targeting the Gag
   gene is shown in SEQ ID NO: 7 or 21 and the sequence of RNA targeting the LTR gene is shown in SEQ ID NO: 9 or 23;
(iii) an RNA targeting the Gag gene and an RNA targeting the LTR gene, wherein the sequence of RNA targeting the Gag
   gene is shown in SEQ ID NO: 8 or 22 and the sequence of RNA targeting the LTR gene is shown in SEQ ID NO: 9 or 23; or
(iv) an RNA targeting the Gag gene and an RNA targeting the Nef gene, wherein the sequence of RNA targeting the Gag
   gene is shown in SEQ ID NO: 7 or 21 and the sequence of RNA targeting the Nef gene is shown in SEQ ID NO: 10 or 24.

### Combination of siRNAs targeting HIV

The fifth aspect of the invention also provides a combination of siRNAs targeting HIV In one embodiment of the invention, the combination comprises at least one of the siRNAs as previously described.

In another embodiment of the invention, the combination comprises at least two of the followings: the siRNA targeting the Gag gene, the siRNA targeting the LTR gene, or the siRNA targeting the Nef gene.

In a preferred embodiment of the invention, the combination of siRNAs targeting HIV comprises:
(iv) a siRNA targeting the Gag gene and a siRNA targeting the LTR gene,
(v) a siRNA targeting the Gag gene and a siRNA targeting the Nef gene, or
(vi) a siRNA targeting the LTR gene and a siRNA targeting the Nef gene.

Further preferably, the sense strand sequence of siRNA targeting the Gag gene is selected from the sequences consisting of 19-23 continuous nucleotides in any one of SEQ ID NOs: 1-3, preferably the sequence shown in any one of SEQ ID NOs: 6-8, and the antisense strand sequence is a sequence reversely complementary to the sense strand. The sense strand sequence of siRNA targeting the LTR gene is selected from the sequences consisting of 19-26 continuous nucleotides in SEQ ID NO: 4, preferably the sequence shown in SEQ ID NO: 9, and the antisense strand sequence is a sequence reversely complementary to the sense strand. The sense strand sequence of siRNA targeting the Nef gene is selected from the sequences consisting of 19-25 continuous nucleotides in SEQ ID NO: 5, preferably the sequence shown in SEQ ID NO: 10, and the antisense strand sequence is a sequence reversely complementary to the sense strand.

In a preferred embodiment of the invention, the combination of siRNAs targeting HIV comprises:
(i) a siRNA targeting the Gag gene and a siRNA targeting the LTR gene, wherein the sense strand sequence of the siRNA
   targeting the Gag gene is shown in SEQ ID NO: 6, the antisense strand sequence is shown in SEQ ID NO: 20, and the sense strand sequence of the siRNA targeting the LTR gene is shown in SEQ ID NO: 9, the antisense strand sequence is shown in SEQ ID NO: 23;
(ii) a siRNA targeting the Gag gene and a siRNA targeting the LTR gene, wherein the sense strand sequence of the
   siRNA targeting the Gag gene is shown in SEQ ID NO: 7, the antisense strand sequence is shown in SEQ ID NO: 21, and the sense strand sequence of siRNA targeting the LTR gene is shown in SEQ ID NO: 9, the antisense strand sequence is shown in SEQ ID NO: 23;
(iii) a siRNA targeting the Gag gene and a siRNA targeting the LTR gene, wherein the sense strand sequence of the siRNA
   targeting the Gag gene is shown in SEQ ID NO: 8, the antisense strand sequence is shown in SEQ ID NO: 22, and the sense strand sequence of siRNA targeting the LTR gene is shown in SEQ ID NO: 9, the antisense strand sequence is shown in SEQ ID NO: 23; or
(iv) a siRNA targeting the Gag gene and a siRNA targeting the Nef gene, wherein the sense strand sequence of the siRNA
   targeting the Gag gene is shown in SEQ ID NO: 7, the antisense strand sequence is shown in SEQ ID NO: 21, and the sense strand sequence of siRNA targeting the Nef gene is shown in SEQ ID NO: 10, the antisense strand sequence is shown in SEQ ID NO: 24.

### Combination of shRNAs targeting HIV or nucleic acids coding therefor

The sixth aspect of the invention also provides a combination of shRNAs targeting HIV or a combination of the nucleic acids coding for the shRNAs targeting HIV

In one specific embodiment of the invention, the combination of shRNAs comprises at least one of the shRNA as previously described. In another specific embodiment of the invention, the combination of the nucleic acids coding for shRNAs comprises at least one of the previously described nucleic acids coding for shRNAs.

In another specific embodiment of the invention, the combination of shRNAs or the nucleic acids coding therefor comprises at least two of the following shRNAs or the nucleic acids coding therefor: a shRNA targeting the Gag gene or the nucleic acid coding therefor, a shRNA targeting the LTR gene or the nucleic acid coding therefor, or a shRNA targeting the Nef gene or the nucleic acid coding therefor.

In a preferred embodiment of the invention, the combination of shRNAs targeting HIV or the nucleic acids coding therefor comprises (i) a shRNA targeting the Gag gene or the nucleic acid coding therefor, and a shRNA targeting the LTR gene or the nucleic acid coding therefor, or (ii) a shRNA targeting the Gag gene or the nucleic acid coding therefor, and a shRNA targeting the Nef gene or the nucleic acid coding therefor, or (iii) a shRNA targeting the LTR gene or the nucleic acid coding therefor, and a shRNA targeting the Nef gene or the nucleic acid coding therefor.

Preferably, the shRNA targeting the Gag gene comprises: (i) the sequences shown in SEQ ID NO: 6 and SEQ ID NO: 20; (ii) the sequences shown in SEQ ID NO: 7 and SEQ ID NO: 21; Or (iii) the sequence shown in SEQ ID NO: 8 and SEQ ID NO: 22. The shRNA targeting the LTR gene comprises the sequences shown in SEQ ID NO: 9 and SEQ ID NO: 23. The shRNA targeting the Nef gene comprises the sequences shown in SEQ ID NO: 10 and SEQ ID NO: 24.

Further preferably, the sequence of the shRNA targeting the Gag gene is shown in any one of SEQ ID NOs: 25-27. The sequence of the shRNA targeting the LTR gene is shown in SEQ ID NO: 28. The sequence of the shRNA targeting the Nef gene is shown in SEQ ID NO: 29.

Further preferably, the sequence of the nucleic acid coding for shRNA targeting the Gag gene is shown in any one of SEQ ID NOs: 11-13. The sequence of the nucleic acid coding for shRNA targeting the LTR gene is shown in SEQ ID NO: 14. The sequence of the nucleic acid coding for shRNA targeting the Nef gene is shown in SEQ ID NO: 15.

In a preferred embodiment of the present invention, the combination of shRNAs targeting HIV or the nucleic acids coding therefor comprises:
(i) a shRNA targeting the Gag gene or the nucleic acid coding therefor, and a shRNA targeting the LTR gene or the
   nucleic acid coding therefor, wherein the shRNA targeting the Gag gene comprises the sequences shown in SEQ ID NO: 6 and SEQ ID NO: 20, and the shRNA targeting the LTR gene comprises the sequences shown in SEQ ID NO: 9 and SEQ ID NO: 23;
(ii) a shRNA targeting the Gag gene or the nucleic acid coding therefor, and a shRNA targeting the LTR gene or the
   nucleic acid coding therefor, wherein the shRNA targeting the Gag gene comprises the sequences shown in SEQ ID NO: 7 and SEQ ID NO: 21, and the shRNA targeting the LTR gene comprises the sequences shown in SEQ ID NO: 9 and SEQ ID NO: 23;
(iii) a shRNA targeting the Gag gene or the nucleic acid coding therefor, and a shRNA targeting the LTR gene or the
   nucleic acid coding therefor, wherein the shRNA targeting the Gag gene comprises the sequences shown in SEQ ID NO: 8 and SEQ ID NO: 22, and the shRNA targeting the LTR gene comprises the sequences shown in SEQ ID NO: 9 and SEQ ID NO: 23; or
(iv) a shRNA targeting the Gag gene or the nucleic acid coding therefor, and a shRNA targeting the Nef gene or the
   nucleic acid coding therefor, wherein the shRNA targeting the Gag gene comprises the sequences shown in SEQ ID NO: 7 and SEQ ID NO: 21, and the shRNA targeting the Nef gene comprises the sequences shown in SEQ ID NO: 10 and SEQ ID NO: 24.

In a preferred embodiment of the present invention, the combination shRNA targeting HIV or the nucleic acids coding therefor comprises:
(i) a shRNA targeting the Gag gene or the nucleic acid coding therefor, and a shRNA targeting the LTR gene or the
   nucleic acid coding therefor, wherein the sequence of the shRNA targeting the Gag gene is shown in SEQ ID NO: 25, and the sequence of the nucleic acid coding therefor is shown in SEQ ID NO: 11, the sequence of the shRNA targeting the LTR gene is shown in SEQ ID NO: 28, and the sequence of the nucleic acid coding therefor is shown in SEQ ID NO: 14;
(ii) a shRNA targeting the Gag gene or the nucleic acid coding therefor, and a shRNA targeting the LTR gene or the
   nucleic acid coding therefor, wherein the sequence of the shRNA targeting the Gag gene is shown in SEQ ID NO: 26, and the sequence of the nucleic acid coding therefor is shown in SEQ ID NO: 12, the sequence of the shRNA targeting the LTR gene is shown in SEQ ID NO: 28, and the sequence of the nucleic acid coding therefor is shown in SEQ ID NO: 14;
(iii) a shRNA targeting the Gag gene or the nucleic acid coding therefor, and a shRNA targeting the LTR gene or the
   nucleic acid coding therefor, wherein the sequence of the shRNA targeting the Gag gene is shown in SEQ ID NO: 27, and the sequence of the nucleic acid coding therefor is shown in SEQ ID NO: 13, the sequence of the shRNA targeting the LTR gene is shown in SEQ ID NO: 28, and the sequence of the nucleic acid coding therefor is shown in SEQ ID NO: 14; or
(iv) a shRNA targeting the Gag gene or the nucleic acid coding therefor, and a shRNA targeting the Nef gene or the
   nucleic acid coding therefor, wherein the sequence of the shRNA targeting the Gag gene is shown in SEQ ID NO: 26, and the sequence of the nucleic acid coding therefor is shown in SEQ ID NO: 12, the sequence of the shRNA targeting the Nef gene is shown in SEQ ID NO: 29, and the sequence of the nucleic acid coding therefor is shown in SEQ ID NO: 15.

### Expression cassette

The seventh aspect of the present invention provides an expression cassette, wherein the expression cassette can express shRNA as described previously.

In one embodiment of the invention, the expression cassette comprises the nucleic acid coding for shRNA or the combination thereof as described previously.

In another preferred embodiment of the invention, the expression cassette comprises a nucleic acid coding for shRNA, or a combination of the nucleic acids coding for shRNAs, wherein the shRNA comprises:
(i) the sequence shown in SEQ ID NO: 6 and SEQ ID NO: 20;
(ii) the sequence shown in SEQ ID NO: 7 and SEQ ID NO: 21;
(iii) the sequence shown in SEQ ID NO: 8 and SEQ ID NO: 22;
(iv) the sequence shown in SEQ ID NO: 9 and SEQ ID NO: 23; or
(v) the sequence shown in SEQ ID NO: 10 and SEQ ID NO: 24.

In a preferred embodiment of the invention, the expression cassette comprises a nucleic acid or a combination thereof which sequence shown in any one of SEQ ID NOs: 11-15.

In the present invention, the expression cassette also contains a promoter. In a preferred embodiment of the invention, the promoter is operably linked with the nucleic acid coding for shRNA as described above. The promoter used in the invention is RNA polymerase II promoter or RNA polymerase III promoter, preferably RNA polymerase III promoter. In a more preferred embodiment of the invention, the RNA polymerase III promoter is H1 promoter, U6 promoter, or 7SK promoter. In another preferred embodiment of the invention, the RNA polymerase II promoter or the RNA polymerase III promoter further comprises any synthetic or modified DNA fragment.

In a preferred embodiment of the invention, the promoter sequence contained in the expression cassette is selected from H1 and/or U6 promoters. In a preferred embodiment of the invention, the expression of shRNA targeting Gag is driven by H1 promoter. Preferably the nucleic acid coding for shRNA targeting Gag is operably linked with H1 promoter. In another preferred embodiment of the invention the expression of shRNA targeting LTR or Nef is driven by U6 promoter. Preferably the nucleic acid coding for shRNA targeting LTR or Nef is operably linked with U6 promoter.

### Vector

The eighth aspect of the invention provides a vector, which contains the RNA or combination thereof, the siRNA or combination thereof, the shRNA or combination thereof, the nucleic acid coding for the shRNA or combination thereof, or the expression cassette, as described previously.

In one embodiment of the invention, the vector comprises the combination of RNAs of the invention, wherein the RNAs of the combination are all contained in the same vector. In another embodiment of the invention, the RNAs of the combination are contained in different vectors.

In one embodiment of the invention, the vector comprises the combination of siRNAs of the invention, wherein the siRNAs of the combination are all contained in the same vector. In another embodiment of the invention, the siRNAs of the combination are contained in different vectors.

In one embodiment of the invention, the vector comprises the combination of the nucleic acids coding for shRNAs of the invention, wherein the nucleic acids coding for shRNAs of the combination are all contained in the same vector. In another embodiment of the invention, the nucleic acids coding for shRNAs of the combination are contained in different vectors.

In one embodiment of the invention, the vector comprises one or more expression cassettes of the invention. In a preferred embodiment of the invention, the one or more expression cassettes are contained in the same vector. In another preferred embodiment of the invention, the one or more expression cassettes are contained in different vectors.

In a preferred embodiment of the invention, the vector comprises:
(i) an expression cassette 1 comprising H1 promoter and a nucleic acid coding for shRNA targeting Gag, wherein the
   sequence of the nucleic acid is shown in any one of SEQ ID NOs: 11-13, and an expression cassette 2 comprising U6 promoter and a nucleic acid coding for shRNA targeting LTR, wherein the sequence of the nucleic acid is shown in SEQ ID NO: 14; or
(ii) an expression cassette 1 comprising H1 promoter and a nucleic acid coding for shRNA targeting Gag, wherein the
   sequence of the nucleic acid is shown in SEQ ID NO: 12, and an expression cassette 2 comprising U6 promoter and a nucleic acid coding for shRNA targeting Nef, wherein the sequence of the nucleic acid is shown in SEQ ID NO: 15.

In one embodiment of the invention, the vector is a DNA vector, a viral vector, a liposome, a nanoparticle, or an exosome.

In a preferred embodiment of the invention, the DNA vector is selected from the group consisting of a DNA plasmid vector, a Molecular coupling binding thereto, and a polymer binding thereto.

In one embodiment of the invention, the viral vector is selected from the group consisting of an adeno-associated viral vector, a lentiviral vector, and an adenoviral vector.

In a preferred embodiment of the invention, the viral vector is a recombinant lentiviral vector.

In a preferred embodiment of the invention, the recombinant lentiviral vector is selected from the group consisting of human immunodeficiency virus (HIV-1 and HIV-2) vector, simian immunodeficiency virus (SIV) vector, feline immunodeficiency virus (FIV) vector, equine infectious anemia virus (EIAV) vector and goat arthritis encephalitis virus (CAEV) vector.

In a preferred embodiment of the invention, the recombinant lentiviral vector is a recombinant simian immunodeficiency virus (SIV) vector.

The eighth aspect of the invention also provides a recombinant virus particle. In a preferred embodiment of the invention, the recombinant simian immunodeficiency virus vector is recombinant simian immunodeficiency virus, also referred to as recombinant simian immunodeficiency virus particle.

In a preferred embodiment of the invention, the recombinant simian immunodeficiency virus or the recombinant simian immunodeficiency virus particle is prepared by using the recombinant simian immunodeficiency gene transfer vector or shuttle plasmid (plasmid carrying foreign gene), packaging vector, rev expression vector and VSV-G expression vector.

In one embodiment of the invention, the recombinant simian immunodeficiency gene transfer vector further comprises RRE, cPPT and WPRE elements. In a preferred embodiment of the present invention, the recombinant simian immunodeficiency gene transfer vector sequentially comprises, from 5 'to 3', 5 'LTR region, RRE, cPPT (central polypurine tract), the expression cassette of the invention, WPRE (woodchuck hepatitis virus posttranscriptional regulatory element) and 3' LTR region.

In one embodiment of the invention, the packaging vector of the present invention (see Chinese patent ZL200680012905.4, derived from the original packaging vector with the amino acid sequence of SEQ ID NO: 27) comprises, *gag* and *pol,* also *vif* and *vpr,* which are referred to as modifying genes, and *tat* and *rev,* which are referred to as regulatory genes. In another embodiment of the present invention, the accessory genes (*vif, vpr, tat*) are removed from the packaging vector, and *rev* is provided in another expression vector, i.e. rev expression vector.

VSV-G expression vector refers to a plasmid expressing VSV-G (Vesical stomatitis virus; VSV) protein. The recombinant SIV viral vector of the invention can be VSV-G pseudotyped. VSV-G pseudotyped refers to modifying the envelope of the vector to contain the surface glycoprotein VSV-G of vesicular stomatitis virus (VSV). After transfection of VSV-G expression vector, the recombinant SIV virus particles coexisted with VSV-G protein during production, and pseudotyped recombinant SIV virus particles were obtained.

By using recombinant lentivirus (e.g. SIV), long-term expression of exogenous genes in cells or living tissues could be achieved, after infecting cells or living tissues.

### Kit

The ninth aspect of the invention provides a kit containing the RNA or combination thereof, the siRNA or combination thereof, the shRNA or combination thereof, the nucleic acid coding for shRNA or combination thereof targeting HIV, the expression cassette, or the vector, as described previously.

### Cell

The tenth aspect of the invention further provides a cell comprising the HIV-targeting RNA or combination thereof, the siRNA or combination thereof, the shRNA or combination thereof, the nucleic acid coding for shRNA or combination thereof, the expression cassette, or the vector, as described previously. The cell of the present invention can be prepared and obtained by conventional methods and techniques well known to those skilled in the art, so long as the obtained cell contains nucleic acids or recombinant vectors as previously described. In a preferred embodiment of the invention, the cell is prepared by infection with recombinant viral vectors as described previously.

In a preferred embodiment of the invention, the cell is derived from HIV receptor cell or peripheral blood mononuclear cell. In a further preferred embodiment of the invention, the cell is derived from lymphocyte. In some embodiments, the cell is derived from T cell. In other embodiments, the cells is derived from naive T cell (Naive T), memory T cell, effector T cell. In other embodiments, the cell is derived from cytotoxic T cell, helper T cell, regulatory T cell. In other embodiments, the T cell is derived from CD4 + T cell, CD8 + T cell. In other embodiments, the cell is derived from NKT cell. In other embodiments, the cell is derived from yδ T cell. In other embodiments, the cell is derived from NK cell. In other embodiments the cell is derived from antigen presenting cell, such as macrophage, dendritic cell.

In a further preferred embodiment of the invention, the cell is derived from stem cell. In some embodiments of the invention, the stem cell includes hematopoietic stem cell (e.g. CD34 + cell) or hematopoietic progenitor cell. In other embodiments of the invention, the stem cell includes memory T stem cell, such as central memory T cell, effector memory T cell, or stem cell-like memory T cell. In another embodiment of the invention, the stem cells can be differentiated into any of the T cells as described above.

In some embodiments, the cell is derived from a patient infected with HIV In some embodiments, the cell is derived from a healthy population.

In another embodiment of the invention, the cell may be isolated (or *in vitro*) cell or cell *in vivo.*

In another embodiment of the invention, the cell is derived from engineered cell, such as TCR-T cell or cell containing CAR molecules.

### Pharmaceutical compositions, pharmaceutical uses and treatment methods

The eleventh aspect of the invention also provides a pharmaceutical composition, wherein the pharmaceutical composition comprises the RNA or combination thereof, the siRNA or combination thereof, the shRNA or combination thereof, the nucleic acid coding for shRNA or combination thereof, the expression cassette, the vector, the cell, as described previously, preferably also comprises a pharmaceutically acceptable carrier.

In one specific embodiment of the invention the pharmaceutical composition further comprises other anti-HIV drugs known in the art. In another specific embodiment of the present invention, other anti-HIV drugs include, but are not limited to, nucleoside inhibitors, HIV vaccines, broad-spectrum neutralizing antibodies, CAR-T cells, etc.

The eleventh aspect of the invention also provides the use of the HIV-targeting RNA or combination thereof, the siRNA or combination thereof, the shRNA or combination thereof, the nucleic acid coding for shRNA or combination thereof, the expression cassette, the vector, the cell or the pharmaceutical composition of the invention, as drugs.

The eleventh aspect of the invention also provides the use of the HIV-targeting RNA or combination thereof, the siRNA or combination thereof, the shRNA or combination thereof, the nucleic acid coding for shRNA or combination thereof, the expression cassette, the vector, the cell or the pharmaceutical composition of the invention in the preparation of drugs for the treatment and/or prevention of HIV infection or AIDS.

The eleventh aspect of the invention also provides a method for treating and/or preventing HIV infection or AIDS, which comprises administering to a patient a therapeutically effective amount of HIV-targeting RNA or combination thereof, siRNA or combination thereof, shRNA or combination thereof, nucleic acid coding for shRNA or combination thereof, expression cassette, recombinant vector, cell or pharmaceutical composition of the invention.

In another aspect, the present invention also provides a method for treating HIV infection or AIDS by using the treatment or prevention methods mentioned above in combination with other methods in the art. In one specific embodiment of the invention, the method for treating and/or preventing HIV infection or AIDS further comprises administering other anti-HIV drugs to a patient. In another specific embodiment of the invention, other anti-HIV drugs include, but not limited to, nucleoside inhibitors, HIV vaccines, broad-spectrum neutralizing antibodies, CAR-T cells, etc.

In the present invention, a therapeutically effective amount refers to the amount of HIV-targeting RNA or combination thereof, siRNA or combination thereof, shRNA or combination thereof, nucleic acid coding for shRNA or combination thereof, expression cassette, vector, cell or pharmaceutical composition that can effectively prevent, relieve or improve disease symptoms or prolong patient survival.

### Method of administration

The twelfth aspect of the invention provides a method of drug administration for the treatment of AIDS, comprising the following steps:
(1) collecting human peripheral blood from patients, isolating peripheral blood mononuclear cells (PBMC) and purifying CD4 + T cells;
(2) infecting the CD4 + T cells in the step (1) with the recombinant viral vector described in the invention, and amplifying the infected CD4 + T cells in vitro; and
(3) the amplified CD4 + T cells were reinfused into patients.

In one embodiment of the invention, CD3/CD28 antibody-coupled magnetic beads are used to activate and amplify CD4 + T cells.

In a preferred embodiment of the invention, the recombinant viral vector infects CD4 + T cells at a multiplicity of infection of 1-20, preferably 20.

The HIV-targeting RNA or combination thereof, siRNA or combination thereof, shRNA or combination thereof, nucleic acid coding for shRNA or combination thereof, expression cassette, recombinant vector, cell or pharmaceutical composition of the invention can significantly inhibit the replication of HIV virus and inhibit the generation of escape mutants for long time.

The invention has the following beneficial effects:
(1) The anti-HIV drug of the invention reduces the replication of the experimental virus strain HIV-1 IIIB to less than 20% of that in virus control group.
(2) The anti-HIV drug of the invention can reduce the replication of the reverse transcriptase inhibitor resistant strain and the protease inhibitor resistant strain to less than 10% of that in virus control group, respectively.
(3) The anti-HIV drug provided by the invention can inhibit the replication of HIV for long time and inhibit the generation of escape mutants to a great extent, and/or
(4) The anti-HIV drug provided by the invention can effectively inhibit HIV virus replication in CD4 + T cells in HIV positive patients, and significantly reduce viral load.

**Table 1: Sequences of exemplary siRNAs and corresponding target sequences on the HIV genome**

| | corresponding target sequences on the HIV genome (5'-3') | siRNA-sense strand (5'-3') | siRNA-antisense strand (5'-3') |
|---|---|---|---|
| Gag | AUCAAUGAGGAAGCUGCAGAAUGG (SEQ ID NO:1) | CAAUGAGGAAGCUGCAGAA (SEQ ID NO. 6) | UUCUGCAGCUUCCUCAUUG (SEQ ID NO:20) |
| Gag | GGGAAGUGACAUAGCAGGAACUACUAG (SEQ ID NO:2) | GUGACAUAGCAGGAACUAC (SEQ ID NO. 7) | GUAGUUCCUGCUAUGUCAC (SEQ ID NO: 21) |
| Gag | UAAAUAAAAUAGUAAGAAUGUAUAGCCCU (SEQ ID NO:3) | AUAGUAAGAAUGUAUAGCC (SEQ ID NO. 8) | GGCUAUACAUUCUUACUAU (SEQ ID NO: 22) |
| LTR | GGAUGGUGCUUCAAGCUAGUACCAGUU (SEQ ID NO:4) | UGCUUCAAGCUAGUACCAG (SEQ ID NO. 9) | CUGGUACUAGCUUGAAGCA (SEQ ID NO: 23) |
| Nef | ACCACACACAAGGCUACUUCCCUGAU (SEQ ID NO:5) | CACAAGGCUACUUCCCUGA (SEQ ID NO.10) | UCAGGGAAGUAGCCUUGUG (SEQ ID NO: 24) |

**Table 2: The sequences of exemplary shRNAs and the nucleic acids coding therefor**

| | the sequences of shRNA (5'-3') | the sequences of the nucleic acid coding for shRNAs (5'-3') |
|---|---|---|
| Gag | CAAUGAGGAAGCUGCAGAAUUCAAGAGAUUCUGCAGC UUCCUCAUUG（SEQ ID NO：25） | CAATGAGGAAGCTGCAGAATTCAAGAGATTCTGCAGCTTC CTCATTG（SEQ ID NO.11） |
| Gag | GUGACAUAGCAGGAACUACUUCAAGAGAGUAGUUCCU GCUAUGUCAC（SEQ ID NO：26） | GTGACATAGCAGGAACTACTTCAAGAGAGTAGTTCCTGCT ATGTCAC（SEQ ID NO.12） |
| Gag | AUAGUAAGAAUGUAUAGCCUUCAAGAGAGGCUAUACAU UCUUACUAU（SEQ ID NO：27） | ATAGTAAGAATGTATAGCCTTCAAGAGAGGCTATACATTCTT ACTAT（SEQ ID NO.13） |
| LTR | UGCUUCAAGCUAGUACCAGUUCAAGAGACUGGUACUAG CUUGAAGCA（SEQ ID NO：28） | TGCTTCAAGCTAGTACCAGTTCAAGAGACTGGTACTAGCTT GAAGCA（SEQ ID NO.14） |
| Nef | CACAAGGCUACUUCCCUGAUUCAAGAGAUCAGGGAAGU AGCCUUGUG（SEQ ID NO：29） | CACAAGGCTACTTCCCTGATTCAAGAGATCAGGGAAGTAG CCTTGTG（SEQ ID NO.15） |

### BRIEF DESCRIPTION OF FIGURES

In order to clearly explain the embodiments of the invention or the technical solutions in the prior art, while a brief description of the figures required for the description of the embodiments or prior art is given below. It is worthy of note that the figures in the following description are only for illustration of the present invention, and other figures can be further obtained based on these figures without creative work for those of skilled in the art.
Fig. 1 shows a map of pGTV-New vector.
Fig. 2 shows the inhibitory effect of recombinant lentivirus single-target drug on HIV
Fig. 3 shows the inhibition curve of recombinant lentivirus SIV-Gag3 and SIV-G3L2 on HIV escape mutants.
Fig. 4 shows the inhibition curve of recombinant lentivirus SIV-Gag2 and SIV-G2N on HIV escape mutants.

### EXAMPLES

The technical solutions in the embodiments of the present invention will be clearly and completely described below in conjunction with the accompanying figures of the present invention. It is worthy of note that the described embodiments are only parts of the present invention, instead of all of them. Based on the embodiments described hereto, all other embodiments that can be obtained without creative work by those skilled in the art should fall within the scope of protection of the present invention.

All the materials or reagents used in the following embodiments are commercially available, unless otherwise specified. The experimental methods in the following embodiments, unless otherwise specified, are all conventional methods in the art.

### Biomaterials

### 1. human immunodeficiency virus (HIV) laboratory standard virus strain

The experimental virus strain HIV-1 IIIB is a laboratory standard virus strain of HIV-1, which has stable replication ability in human T lymphocyte line H9, and is a widely used model for in vitro HIV drug efficacy evaluation, either in the literature or practice. The HIV-1 IIIB virus strain of the invention is from the Medical Research Council AIDS Reagent Project of the United Kingdom (UK), and is propagated and preserved with the titer of 5×10⁴ TCID 50/ml, and stored below-70°C.

### 2. Drug-resistant strains of human immunodeficiency virus (HIV)

The first-line and second-line drugs for treating HIV virus infection include reverse transcriptase inhibitor, protease inhibitor, integrase inhibitor and fusion inhibitor. The mutants resistant to reverse transcriptase inhibitor and protease inhibitor are the major HIV drug-resistant mutant strains. These two drug-resistant strains are selected as experimental virus strain in the present invention. The HIV reverse transcriptase inhibitor resistant strain MT2-010 and the HIV protease inhibitor resistant strain MT2-884 used in examples are prepared and obtained according to the methods described in patent ZL201710172266.8 and ZL201210533551.5, respectively.

### 3. H9 cells and MT2 cells are purchased from Otwo Biotech (Shenzhen) INC., 2 × 10⁶cells/tube. The cells were resuscitated and subcultured before use.

### EXAMPLE 1 Construction of shuttle plasmid carrying nucleic acid coding for shRNA targeting HIV genome

### 1. Construction of intermediate vector pGTV-new:

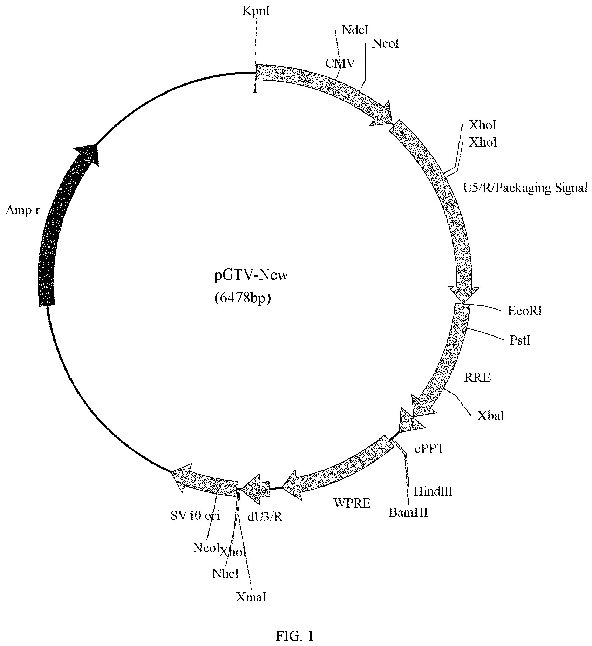

(1) Vector digestion: The pGTV-PEDF vector was digested by EcoR I and NheI (the preparation method of the vector is described in section 1-1 of Example 1 of patent application CN200680012905.4, which is incorporated herein by reference in its entirety), and the vector fragment of about 5.5 kb was recovered.
(2) Fragment 1 amplification: primer 1: ATCGAATTCC CGTTTGTGCT AG (SEQ ID NO. 16) and primer 2: AAGCTTGCGGGATCCCCGCG GCTCTAAAAT TTA (SEQ ID NO. 17) were synthesized. pGTV-PEDF was used as template, and fragment 1 of about 500bp was amplified and recovered.
(3) Fragment 2 amplification: primer 3: AGCGCAGCGA GTCAGTGAGC G (SEQ ID NO. 18) and primer 4: GGATCC CGC AAGCTT AG ATCCGCACTT TTTAAAAG (SEQ ID NO. 19) were synthesized. pGTV-PEDF was used as template, and fragment 2 of about 500bp was amplified and recovered.
(4) Overlapping PCR: The recovered fragment 1 and fragment 2 were mixed and then used as template. A fragment of about 1000bp was amplified by primer 1 and primer 4. After recovery and purification, the fragment was digested by EcoR I and NheI and then recovered.
(5) The vector fragment obtained in step (1) and the recovered fragment obtained in step (4) were ligated with each other to construct an intermediate vector, named as pGTV-New, the map of which is shown in Fig 1.

### 2. Construction of shuttle plasmid carrying nucleic acid coding for shRNA targeting a single-target in HIV genome

H1-Gag1 sequence fragment, H1-Gag2 sequence fragment, H1-Gag3 sequence fragment, U6-LTR2 sequence fragment and U6-Nef sequence fragment were synthesized respectively (the synthetic Gag, LTR and Nef sequences used in the vector construction process of the invention are DNA sequences coding for shRNA targeting the genes in HIV genome). HindIII and BamHI restriction sites were introduced on two ends of the fragments during the process of synthesis, respectively. H1 and U6 are promoters. The nucleic acids coding for shRNA targeting Gag gene in HIV genome were respectively referred to as Gag1, Gag2 and Gag3, the nucleic acid coding for shRNA targeting LTR gene in HIV genome was referred to as LTR2, and the nucleic acid coding for shRNA targeting Nef gene in HIV genome was referred to as Nef, as follows:
CAATGAGGAAGCTGCAGAATTCAAGAGATTCTGCAGCTTCCTCATTG (SEQ ID NO.11) (refer to as Gag1);
GTGACATAGCAGGAACTACTTCAAGAGAGTAGTTCCTGCTATGTCAC (SEQ ID NO.12) (refer to as Gag2);
ATAGTAAGAATGTATAGCCTTCAAGAGAGGCTATACATTCTTACTAT (SEQ ID NO.13) (refer to as Gag3);
TGCTTCAAGCTAGTACCAGTTCAAGAGACTGGTACTAGCTTGAAGCA (SEQ ID NO.14) (refer to as LTR2);
CACAAGGCTACTTCCCTGATTCAAGAGATCAGGGAAGTAGCCTTGTG (SEQ ID NO.15) (refer to as Nef).

The above synthesized H1-Gag3, H1-Gag2, H1-Gag1, H1-LTR2 and H1-Nef fragments were digested by HindIII and BamHI, respectively, and then ligated respectively with pGTV-new vectors which were digested by the same enzymes, thus obtaining a series of shuttle plasmids of pGTV-Gag3, pGTV-Gag2, pGTV-Gag1, pGTV-LTR2 and pGTV-Nef, each of which carries a nucleic acid coding for shRNA for single-target in HIV genome, respectively. The shuttle plasmids were sequenced and verified to be correct.

### 3. Construction of shuttle plasmid carrying nucleic acids coding for shRNA targeting dual-targets in HIV genome

H1-Gag3-U6-LTR2 sequence fragment, H1-Gag2-U6-LTR2 sequence fragment, H1-Gag1-U6-LTR2 sequence fragment and H1-Gag2-U6-Nef sequence fragment were synthesized respectively. HindIII and BamHI restriction sites were introduced on two ends of the fragments during the process of synthesis, respectively. H1 and U6 were promoters, and there was a spacer sequence between H1 expression frame and U6 expression frame.

The above synthesized H1-Gag3-U6-LTR2, H1-Gag2-U6-LTR2, H1-Gag1-U6-LTR2, H1-Gag2-U6-Nef sequence framents were digested by HindIII and BamHI, respectively, and then ligated respectively with pGTV-new vectors which were digested by the same enzymes, thus obtaining a series of shuttle plasmids of pGTV-Gag3-LTR2, pGTV-Gag2-LTR2, pGTV-Gag1-LTR2 and pGTV-Gag2-Nef, each of which carryies nucleic acids coding for shRNAs for dual-targets in HIV genome, respectively. The above shuttle plasmids were sequenced and verified to be correct.

### 4. Preparation of recombinant lentivirus SIV

### (1) Packaging and purification of recombinant lentivirus SIV

The methods of constructing packaging vector, rev expression vector and VSV-G expression vector are described in Embodiment 1of Chinese patent ZL200680012905.4.

The 293T cells (ATCC, CRL-11268) were inoculated with 9×10⁶ cells per T225 culture flask (Coring, Cat # 431082), and cultured in 20ml D-MEM medium (Thermofisher, Cat # 11995-065) containing 10% fetal bovine serum for 48 hours. After culture, the medium was replaced with 10ml preheated OPTI-MEM medium (Thermofisher, Cat # 31985-070), and the cells were ready to be transfected.

The transfection system was prepared separately for each T225 culture flask. First, 60 µ g of the shuttle plasmid obtained in Example 1 was dissolved in 2.25 ml of HBSS buffer together with 30 µ g of packaging vector, 12 µ g of rev expression vector, and 12 µ g of VSV-G expression vector, obtaining plasmid mixture. Then, 167 nM of CaCl₂ solution was prepared. 2.25 ml of the plasmid mixture described above was added into 2.25 ml of the CaC1₂ solution above. The solution was mixed immediately by vortex for 5s, and incubated for 10-15 min at room temperature to obtain about 4.5 ml of solution containing plasmid DNA-CaCl₂ complex. Subsequently, said solution was added into the above-mentioned culture flasks containing cells and incubated in incubator at 37°C, 5% CO₂ for 3 hours. Then, each flask was supplemented with DMEM medium containing 20% FBS, with the final concentration of FBS being 10%, and incubated overnight in incubator at 37°C, 5% CO₂.

20 hours after transfection, the medium in each T225 culture flask was replaced with 20 ml of fresh preheated DMEM medium. 48 hours after transfection, the supernatant was recovered and filtered with a 0.45 µ m filter to obtain a primary filtrate, which was then concentrated using a high-speed centrifuge. Specifically, the primary filtrate was placed in a high-speed centrifuge tube and centrifuged at 4°C, 40000 g for 2 hours. After the supernatant was completely removed from the centrifuge tubes, DPBS was added to each centrifuge tube to cover the precipitate, and the precipitate was subsequently resuspended to obtain the recombinant lentivirus SIV concentrate. After subpackaging, the virus concentrate could be used directly or stored at -80 °C.

### (2) Determination of the Genomic titer of recombinant lentivirus SIV

The genomic RNA of the virus was extracted and then reverse transcribed into cDNA. The obtained cDNA was used as a template for real-time quantitative PCR. For steps in details, see the titer determination method described in section 4-3 of embodiment 4 in Chinese patent ZL201210078288.5 for determination of genomic titer of the recombinant lentivirus SIV (the patent is incorporated herein by reference in its entirety). After determination, the genomic titer of all the recombinant lentivirus SIVs packaged in the example was 3 × 10¹⁰Vg/ml.

In the context of the application, recombinant lentiviral vector packaged by the shuttle plasmids pGTV-Gag3, pGTV-Gag2, pGTV-Gag1, pGTV-LTR2, pGTV-Nef, pGTV-Gag3-LTR2, pGTV-Gag2-LTR2, pGTV-Gag1-LTR2 or pGTV-Gag2-Nef and the packaging vectors, the rev expression vectors, the VSV-G expression vectors, were referred to as recombinant lentivirus SIV-Gag3, SIV-Gag2, SIV-Gag1, SIV-LTR2, SIV-Nef, SIV-G3L2, SIV-G2L2, SIV-G1L2, or SIV-G2N, respectively.

### EXAMPLE 2 Inhibitory effect of recombinant lentivirus single-target drug on HIV

### (1) Administration on H9 cells:

The H9 cells were resuscitated and subcultured in complete medium (RPMI-1640 + 10% FBS + PS). The cells were resuspended in OPTI-MEM medium, counted and adjusted to a concentration of 5 × 10⁵ cells/ml, and inoculated in 6-well plates with 2ml per well. The experimental groups SIV-Gag3, SIV-Gag2, SIV-Gag1, SIV-LTR2 or SIV-Nef, SIV-EGFP empty vector control group and blank control group (no HIV-1 IIIB infection, and recombinant lentivirus drug administration) were set on the 6-well plates. The above recombinant lentiviruses were added to the 6-well plates with the multiplicity of infection (MOI) of 2000, mixed, and cultured at 37°C, 5% CO₂ for 24 hours. Each well was supplemented with 4ml complete medium and cultured at 37°C, 5% CO₂ for 72 hours.

### (2) Virus Challenge

5 ml of supernatant from each well of 6-well plates was discarded and another 5 ml of fresh complete medium was added. Cells were diluted and inoculated in 96-well plates at 1×10⁴ cells/well. 0.5 ml of HIV-1 IIIB was diluted with 5.75 ml of complete medium and mixed. Then 100 µ 1 of virus diluent was added to each well of cells, except for the blank control group, mixed and incubated at 37°C and 5% CO₂. Half volume of liquid was changed on day 2 and day 4 after HIV-1 IIIB infection, respectively. 150 µ l of sample was taken from each well on day 7 after infection, sealed and heated at 56°C for 30 minutes. After the HIV viruses were thermally inactivated, the sample was stored at-20°C, ready for ELISA detection.

### (3) ELISA detection for Sample

HIV-1 P24 antigen detection kit (96T) (Beijing Jinboyi Biotechnology Co., Ltd.) was used for ELISA detection. The OD Value was measured with a microplate reader. Then average value, and relative concentration was calculated to determine the antiviral activity of the recombinant lentiviruses against HIV-1 IIIB in H9 cells.

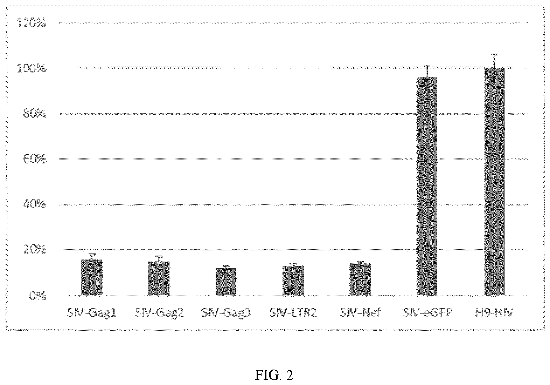

The result was shown in Fig. 2. All five recombinant lentivirus single-target drugs showed significant inhibitory effects on the replication of HIV IIIB experimental strains in H9 cells.

### Example 3: Efficacy of Recombinant Lentivirus Dual-Targets Drugs on Experimental Strains

### (1) Administration on H9 cells:

The H9 cells were resuscitated and subcultured in complete medium (RPMI-1640 + 10% FBS + PS). The cells were resuspended in OPTI-MEM medium, counted and adjusted to a concentration of 5 × 10⁵ cells/ml, and inoculated in 6-well plates with 2ml per well. The experimental groups SIV-G3L2, SIV-G2L2, SIV-G1L2, or SIV-G2N, SIV-EGFP empty vector control group, positive drug control group (Efavirenz, EFV, Shanghai Desano Pharmaceuticals Co., Ltd., Concentration 1 µM), no-drug control group (HIV-1 IIIB infection, and no recombinant lentivirus drug administration) and blank control group (no HIV-1 IIIB infection, and no lentivirus drug administration) were set on the 6-well plates. The recombinant lentiviruses were added to the 6-well plates at five doses with the multiplicity of infection (MOI) of 125, 250, 500, 1000, and 2000, and the added volumes were 2.1 µL, 4.2 µL, 8.4 µL, 16.7 µL, 33.3 µL, respectively, with one well for each dose. The recombinant lentivirus SIV-EGFP with MOI of 2000 was added as an empty vector control, the volume was 33.3 µL. Mixed, and cultured at 37 °C and 5% CO₂ for 24 hours. Each well was supplemented with 4ml complete medium and cultured at 37°C, 5% CO₂ for 72 hours.

### (2) Virus Challenge

5 ml of supernatant from each well of 6-well plates was discarded and another 5 ml of fresh complete medium was added, the cells were diluted and inoculated in 96-well plates at 1×10⁴ cells/well. 0.5 ml of HIV-1 IIIB was diluted with 5.75 ml of complete medium and mixed. Then 100 µ l of virus diluent was added to each well of cells, except for the blank control group, mixed and incubated at 37°C and 5% CO₂. Half volume of liquid was changed on day 2 and day 4 after HIV-1 IIIB infection, respectively. 150 µ l of sample was taken from each well on day 7 after infection, sealed and heated at 56°C for 30 minutes. After the HIV viruses were thermally inactivated, the sample was stored at-20°C, ready for ELISA detection.

### (3) ELISA detection for Sample

HIV-1 P24 antigen detection kit (96T) (Beijing Jinboyi Biotechnology Co., Ltd.) was used for ELISA detection. The OD Value was measured with a microplate reader. Then average value, and relative concentration was calculated to determine the antiviral activity of the recombinant lentiviruses against HIV-1 IIIB in H9 cells.

**Table 3 The antiviral activity of the tested drug SIV-G3L2 against HIV-1 IIIB in H9 cells**

| Cell and virus strain | Tested drug | Average concentration (pg/ml) | Relative concentration(%) |
|---|---|---|---|
| H9 + HIV-1 IIIB | SIV-G3L2 | 2007.833 | 1094 |
| | MOI=2000 | | |
| H9 + HIV-1 IIIB | SIV-G3L2 | 4238.1 | 23.10 |
| | MOI=1000 | | |
| H9 + HIV-1 IIIB | SIV-G3L2 | 7087.067 | 38.63 |
| | MOI=500 | | |
| H9 + HIV-1 IIIB | SIV-G3L2 | 12493.8 | 68.10 |
| | MOI=250 | | |
| H9 + HIV-1 IIIB | SIV-G3L2 | 13834.8 | 75.42 |
| | MOI=125 | | |
| H9 + HIV-1 IIIB | SIV-EGFP | 19039.6 | 103.79 |
| | MOI=2000 | | |
| H9 + HIV-1 IIIB | None | 18344.4 | 10000 |
| | | | |
| H9 + HIV-1 IIIB | EFV | 10 | 0.05 |
| H9 | None | 10 | 0.05 |

**Table 4 The antiviral activity of the tested drug SIV-G2L2 against HIV-1 IIIB in H9 cells**

| Cell and virus strain | Tested drug | Average concentration of P24 (pg/ml) | Relative concentration %) |
|---|---|---|---|
| H9 + HIV-1 IIIB | SIV-G2L2 | 3166.02 | 12.32 |
| | MOI=2000 | | |
| H9 + HIV-1 IIIB | SIV-G2L2 | 6774.05 | 26.36 |
| | MOI=1000 | | |
| H9 + HIV-1 IIIB | SIV-G2L2 | 10428.33 | 40.58 |
| | MOI=500 | | |
| H9 + HIV-1 IIIB | SIV-G2L2 | 19435.55 | 75.63 |
| | MOI=250 | | |
| H9 + HIV-1 IIIB | SIV-G2L2 | 23192.63 | 90.25 |
| | MOI=125 | | |
| H9 + HIV-1 IIIB | SIV-eGFP | 25945.6 | 100.96 |
| | MOI=2000 | | |
| H9 + HIV-1 IIIB | None | 25698.2 | 100 |
| H9 + HIV-1 IIIB | EFV | <10 | 0.05 |
| H9 | None | <10 | 0.05 |

**Table 5 The antiviral activity of the tested drug SIV-G1L2 against HIV-1 IIIB in H9 cells**

| Cell and virus strain | drug | Average concentration of P24 (pg/ml) | Relative concentration(%) |
|---|---|---|---|
| H9 + HIV-1 IIIB | SIV-G1L2 | 2954.78 | 1501 |
| | MOI=2000 | | |
| H9 + HIV-1 IIIB | SIV-G1L2 | 4968.26 | 25.24 |
| | MOI=1000 | | |
| H9 + HIV-1 IIIB | SIV-G1L2 | 8865.42 | 45.04 |
| | MOI=500 | | |
| H9 + HIV-1 IIIB | SIV-G1L2 | 15597.6 | 79.23 |
| | MOI=250 | | |
| H9 + HIV-1 IIIB | SIV-G1L2 | 19963.57 | 101.41 |
| | MOI=125 | | |
| H9 + HIV-1 IIIB | SIV-eGFP | 18963.25 | 96.33 |
| | MOI=2000 | | |
| H9 + HIV-1 IIIB | None | 19685.41 | 100 |
| H9 + HIV-1 IIIB | EFV | <10 | 0.05 |
| H9 | None | <10 | 0.05 |

**Table 6 The antiviral activity of the tested drug SIV-G2N against HIV-1 IIIB in H9 cells**

| Cell and virus strain | drug | Average concentration of P24 (pg/ml) | Relative concentration(%) |
|---|---|---|---|
| H9 + HIV-1 IIIB | SIV-G2N | 2653.78 | 13.28 |
| | MOI=2000 | | |
| H9 + HIV-1 IIIB | SIV-G2N | 6085.96 | 30.45 |
| | MOI=1000 | | |
| H9 + HIV-1 IIIB | SIV-G2N | 8607.63 | 43.06 |
| | MOI=500 | | |
| H9 + HIV-1 IIIB | SIV-G2N | 14235.89 | 71.23 |
| | MOI=250 | | |
| H9 + HIV-1 IIIB | SIV-G2N | 16798.19 | 84.47 |
| | MOI=125 | | |
| H9 + HIV-1 IIIB | SIV-eGFP | 19397.13 | 97.05 |
| | MOI=2000 | | |
| H9 + HIV-1 IIIB | None | 19986.74 | 100 |
| H9 + HIV-1 IIIB | EFV | <10 | 0.05 |
| H9 | None | <10 | 0.05 |

As shown in Table 3 to Table 6, the recombinant lentivirus SIV-G3L2, SIV-G2L2, SIV-G1L2 or SIV-G2N all showed inhibitory effects on the replication of HIV virus in H9 cells, with obvious dose-effect relationships. With the increase of the dose of the recombinant lentivirus SIV-G3L2, SIV-G2L2, SIV-G1L2 or SIV-G2N, their inhibitory effect on HIV replication also increased. In high dose group (MOI=2000), recombinant lentivirus SIV-G3L2, SIV-G2L2, SIV-G1L2 or SIV-G2N reduced the replication of HIV to 10.94%, 12.32%, 15.01% or 13.28% of that in the no-drug control group, respectively, indicating that the recombinant lentivirus SIV-G3L2, SIV-G2L2, SIV-G1L2 or SIV-G2N could effectively inhibit the replication of HIV-1 IIIB.

### EXAMPLE 4 Drug efficacy of recombinant lentivirus dual-targets drugs against reverse transcriptase inhibitor resistant strains

The experimental design of example 4 was the same as that of Example 3, except that the tested strain was a reverse transcriptase inhibitor resistant strain MT2-010, and the infected cells were MT2 cells. Firstly, cell culture and recombinant lentivirus infection: the experimental group SIV-G3L2 or SIV-G2N, SIV-EGFP empty vector control group, positive drug control group (Efavirenz, EFV, Shanghai Desano Pharmaceuticals Co., Ltd., Concentration 1 µM), no drug control group (MT2-010 infection, and no recombinant lentivirus drug administration), and a blank control group were set on the 6-well plates. The recombinant lentiviruses were added to the 6-well plates with the multiplicity of infection (MOI) of 125, 250, 500, 1000 and 2000, respectively, and the recombinant lentivirus SIV-EGFP with MOI of 2000 was added as an empty vector control. Mixed and cultured.

Then virus challenge: the MT2 cells were diluted and inoculated in 96-well plates at 1×10⁴ cells/well. The reverse transcriptase inhibitor resistant strain MT2-010 was diluted with complete culture medium, and mixed. The virus diluent was added into each well of cells, except for the blank control group. Mixed and cultured. The sampling was performed as described in Example 3, and ELISA detection was performed by using the above-mentioned kit. The OD value was measured with a microplate reader. Then average value, and relative concentration was calculated to determine the antiviral activity of the recombinant lentiviruses against MT2-010 in MT2 cells.

**Table 7 Antiviral activity of recombinant lentivirus SIV-G3L2 against reverse transcriptase inhibitor resistant strain MT2-010**

| Cell and virus strain | Sample | Average concentration (pg/ml) | Relative concentration(%) |
|---|---|---|---|
| MT2+MT2-010 | SIV-G3L2 | 2867.25 | 9.14 |
| | MOI=2000 | | |
| MT2+MT2-010 | SIV-G3L2 | 5795.767 | 18.48 |
| | MOI=1000 | | |
| MT2+MT2-010 | SIV-G3L2 | 12456.7 | 3973 |
| | MOI=500 | | |
| MT2+MT2-010 | SIV-G3L2 | 18488.1 | 58.96 |
| | MOI=250 | | |
| MT2+MT2-010 | SIV-G3L2 | 23877.8 | 76.15 |
| | MOI=125 | | |
| MT2+MT2-010 | SIV-eGFP | 27584.67 | 87.97 |
| | MOI=2000 | | |
| MT2+MT2-010 | None | 31356.67 | 10000 |
| MT2+MT2-010 | EFV | 52.69 | 0.17 |
| MT2 | None | 0 | 0.00 |

As shown in Table 7, the recombinant lentivirus SIV-G3L2 showed inhibitory effect on the replication of HIV reverse transcriptase inhibitor resistant strain in MT2 cells, with a significant dose-effect relationship. With the increase of the dose of the recombinant lentivirus SIV-G3L2, its inhibitory effect on HIV replication also increased. In high dose group (MOI=2000), the recombinant lentivirus SIV-G3L2 reduced the replication of HIV to 9.14 ± 2.09% of that in the no-drug control group, indicating that the recombinant lentivirus SIV-G3L2 could effectively inhibit the replication of the reverse transcriptase inhibitor resistant strain.

**Table 8 Antiviral activity of recombinant lentivirus SIV-G2N against reverse transcriptase inhibitor resistant strain MT2-010**

| Cell and virus strain | Sample | Average concentration (pg/ml) | Relative concentration(%) |
|---|---|---|---|
| MT2+MT2-010 | SIV-G2N | 3273.98 | 11.31 |
| | MOI=2000 | | |
| MT2+MT2-010 | SIV-G2N | 6147.65 | 21.24 |
| | MOI=1000 | | |
| MT2+MT2-010 | SIV-G2N | 12955.2 | 44.77 |
| | MOI=500 | | |
| MT2+MT2-010 | SIV-G2N | 15991.68 | 55.26 |
| | MOI=250 | | |
| MT2+MT2-010 | SIV-G2N | 21369.7 | 73.84 |
| | MOI=125 | | |
| MT2+MT2-010 | SIV-EGFP | 27698.36 | 95.71 |
| | MOI=2000 | | |
| MT2+MT2-010 | None | 28937.06 | 10000 |
| MT2+MT2-010 | EFV | 59.36 | 0.21 |
| MT2 | None | 0 | 0.00 |

As shown in Table 8, the recombinant lentivirus SIV-G2N also showed a strong inhibitory effect on the replication of HIV reverse transcriptase inhibitor resistant strain in MT2 cells, with a significant dose-effect relationship. With the increase of the dose of the recombinant lentivirus SIV-G2N, its inhibitory effect on HIV replication also increased. In high dose group (MOI=2000), the recombinant lentivirus SIV-G2N reduced the replication of HIV to 11.31 + 1.34% of that in the no-drug control group, indicating that the recombinant lentivirus SIV-G2N could effectively inhibit the replication of the reverse transcriptase inhibitor resistant strain.

### EXAMPLE 5 Drug efficacy of recombinant lentivirus drugs against Protease inhibitor resistant strains

The experimental method of example 5 was the same as Step 1 of Example 3, except that the tested strain was a protease inhibitor resistant strain MT2-884, and the infected cells were MT2 cells. Firstly, cell culture and recombinant lentivirus SIV-G3L2 or SIV-G2N infection: Cells were cultured in 6-well plates, and the experimental group SIV-G3L2 or SIV-G2N, SIV-EGFP empty vector control group, positive drug control group (Efavirenz, EFV, Shanghai Desano Pharmaceuticals Co., Ltd., Concentration 1 µM), no drug control group (MT2-884 infection, and no recombinant lentivirus drug administration), and a blank control group were set on the 6-well plates. The recombinant lentiviruses were added to the 6-well plates with the multiplicity of infection (MOI) of 125, 250, 500, 1000 and 2000, respectively, and the recombinant lentivirus SIV-EGFP with MOI of 2000 was added as an empty vector control. Mixed and cultured.

Then virus challenge: the MT2 cells were diluted and inoculated in 96-well plates at 1×10⁴ cells/well. The protease inhibitor resistant strain MT2-884 was diluted with complete culture medium, the virus diluent was added into each well of cells except for the blank control group. Mixed and cultured. The time sampling was performed as described in Example 3, and ELISA detection was performed by using the above-mentioned kit. The OD value was measured with a microplate reader. then average value, and relative concentration was calculated to determine the antiviral activity of the recombinant lentiviruses against MT2-884 in MT2 cells.

**Table 9 Antiviral activity of recombinant lentivirus SIV-G3L2 against protease inhibitor resistant strain MT2-884**

| Cell and virus strain | Sample | Average concentration(pg/ml) | Relative concentration(%) |
|---|---|---|---|
| MT2+MT2-884 | SIV-G3L2 | 961.32 | 9.72 |
| | MOI2000 | | |
| MT2+MT2-884 | SIV-G3L2 | 193734 | 19.58 |
| | MOI 1000 | | |
| MT2+MT2-884 | SIV-G3L2 | 4698.867 | 47.49 |
| | MOI500 | | |
| MT2+MT2-884 | SIV-G3L2 | 6883.733 | 69.58 |
| | MOI250 | | |
| MT2+MT2-884 | SIV-G3L2 | 11084.27 | 112.04 |
| | MOI125 | | |
| MT2+MT2-884 | SIV-eGFP | 11021 | 111.40 |
| MT2+MT2-884 | None | 9893.5 | 10000 |
| MT2+MT2-884 | EFV | 50 | 0.51 |
| MT2 | None | 0 | 0.00 |

As shown in Table 9, the recombinant lentivirus SIV-G3L2 showed inhibitory effect on the replication of HIV protease inhibitor resistant strain in MT2 cells, with a significant dose-effect relationship. With the increase of the dose of the recombinant lentivirus SIV-G3L2, its inhibitory effect on HIV replication also increased. In high dose group (MOI=2000), the recombinant lentivirus SIV-G3L2 reduced the replication of HIV to 9.72 + 0.70% of that in the no-drug control group, indicating that the recombinant lentivirus SIV-G3L2 could effectively inhibit the replication of the HIV protease inhibitor resistant strain.

The recombinant lentivirus SIV-G2N showed inhibitory effect on the replication of HIV protease inhibitor resistant strain in MT2 cells, with a significant dose-effect relationship. With the increase the dose of the recombinant lentivirus SIV-G2N, its inhibitory effect on HIV replication also increased. In high dose group (MOI=2000), the recombinant lentivirus SIV-G2N reduced the replication of HIV to 9.02 + 0.70% of that in the no-drug control group, indicating that the recombinant lentivirus SIV-G2N could effectively inhibit the replication of the HIV protease inhibitor resistant strain (data not shown).

### EXAMPLE 6 Inhibition of HIV escape mutants by recombinant lentivirus drugs

H9 cells were infected with the lentiviral vector drug SIV-G3L2 or SIV-G2N prepared in Example 1. The cells were infected with recombinant lentivirus SIV-EGFP empty vector and recombinant lentivirus single-target drug SIV-Gag3 or SIV-Gag2, respectively, as control groups. The virus infection titer in all group was 3×10¹⁰ Vg/ml. H9 cells were inoculated in six-well plates, with 1×10⁶ cells/well. At the same time, 33.3 µ l of each above recombinant lentivirus was added to each well, respectively, with MOI=2000. 24 hours after infection, 2ml complete culture medium was added to each well. The cells were cultured for another 48 hours, and then diluted as monoclonal culture.

DNA of H9 cells infected by recombinant lentivirus was extracted and amplified by PCR, and the integration of virus gene was detected through band size. Five monoclonal cells were randomly selected from each of the samples infected by the above three viruses respectively, and HIV escape mutant inhibition experiment was carried out. The infected cell strain was named as H9-EGFP-1, H9-EGFP-2, H9-EGFP-3, H9-EGFP-4, H9-EGFP-5, H9-Gag3-1, H9-Gag3-2, H9-Gag3-3, H9-Gag3-4, H9-Gag3-5, H9-G3L2-1, H9-G3L2-2, H9-G3L2-3, H9-G3L2-4, or H9-G3L2-5, respectively.

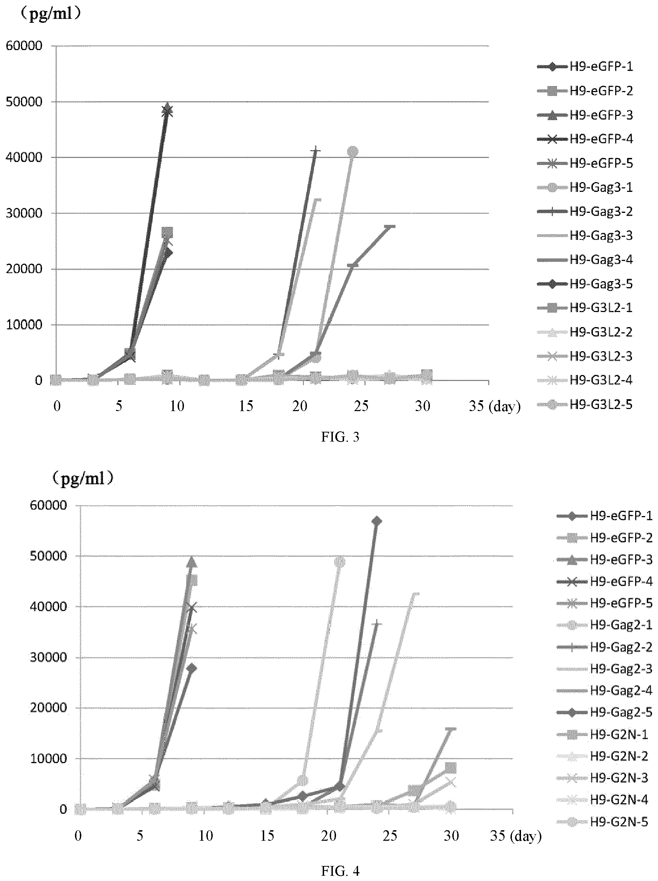

In the above 15 monoclonal cell strains culture systems, HIV-1 IIIB was added with MOI = 0.002 TCID50 to each system, and co-cultured for 30 days, with cell passaging and P24 in supernatant being detected every 3 days. When the concentration of p24 in the supernatant of a cell strain culture increased significantly, indicating that the recombinant lentivirus drug fails to inhibit HIV, this cell strain culture was supposed to stop. The detection results in different cell strains were shown in Tables 10-11 and FIGs. 3-4.

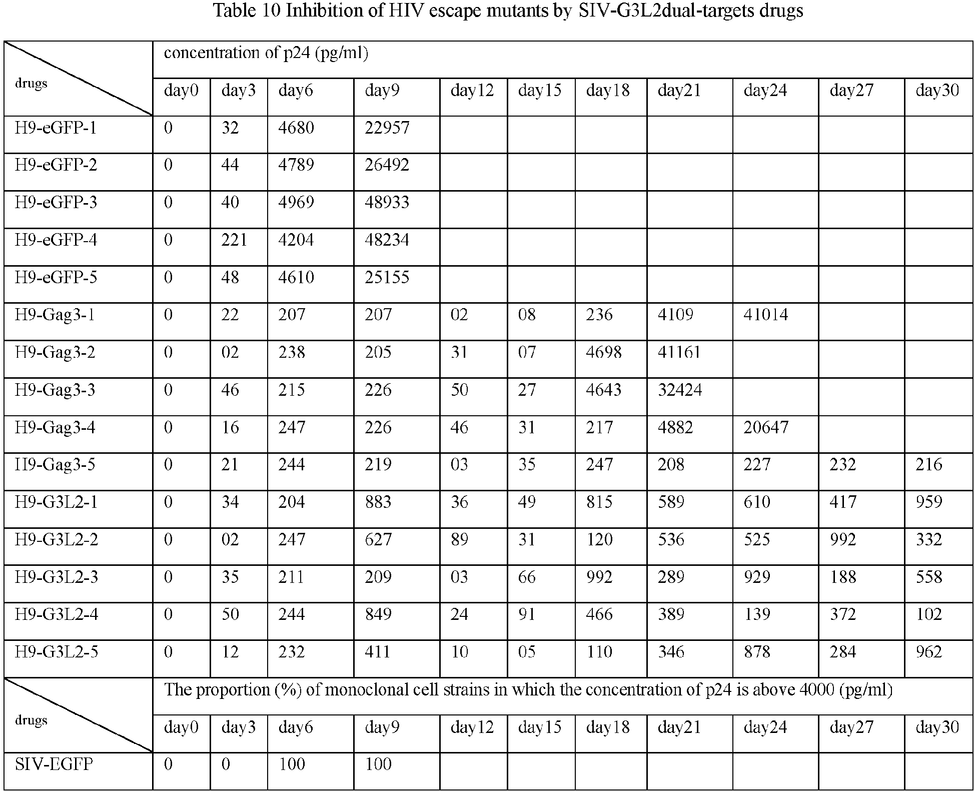

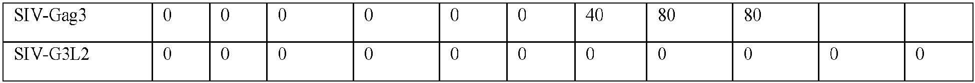

As shown in Table 10 and Fig. 3, the recombinant lentivirus SIV-eGFP empty vector did not inhibit the replication of HIV virus, and a significant replication of HIV was detected obviously after culture for 3-6 days. After infected with recombinant lentivirus single-target drug SIV-Gag3, the H9 cells showed resistance to HIV, however, in four monoclonal cell strains, a significant replication of HIV was also detected on days 18-21, indicating that the drug-resistant mutants to recombinant lentivirus single-target drug SIV-Gag3 occurred at this time. After H9 cells were infected with recombinant lentivirus dual-targets drug SIV-G3L2, no significant replication of HIV was detected in all five monoclonal cell strains during the experimental culture time range (30 days), which indicated that recombinant lentivirus dual-targets drug SIV-G3L2 not only significantly inhibited the replication of HIV, but also inhibited the generation of drug-resistant mutants to a great extent.

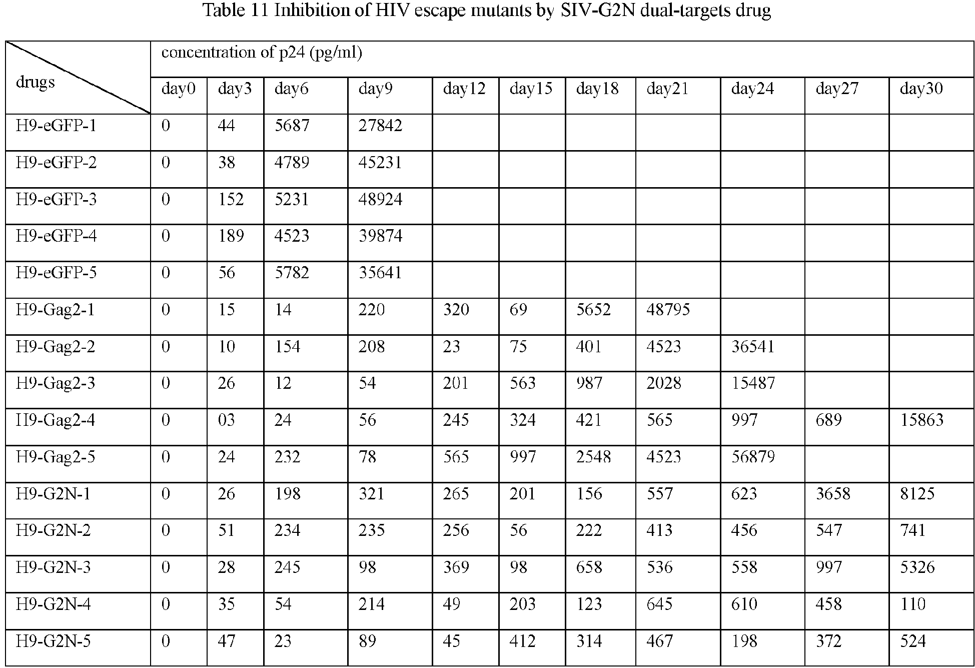

As shown in Table 11 and Fig. 4, the recombinant lentivirus SIV-EGFP empty vector did not inhibit the replication of HIV virus, and a significant replication of HIV was detected obviously after culture for 3-6 days. After infected with recombinant lentivirus single-target drug SIV-Gag2, the H9 cells showed resistance to HIV, however, in all five monoclonal cell strains a significant replication of HIV was also detected on days 18-30, indicating that the drug-resistant mutants to recombinant lentivirus single-target drug SIV-Gag2 occurred at this time. After H9 cells were infected with recombinant lentivirus dual-targets drug SIV-G2N, no significant replication of HIV was detected in three monoclonal cell strains during the experimental culture time range (30 days), and a small amount of replication was detected in two monoclonal cell strains on days 27-30, which indicated that recombinant lentivirus dual-targets drug SIV-G2N not only significantly inhibited the replication of HIV, but also inhibited the generation of drug-resistant mutants to a great extent.

### Example 7 Inhibitory effect of recombinant lentivirus drugs on HIV virus replication in CD4 + T cells of HIV patients

### 1. Isolation of PBMC cells

Using Ficoll-Paque PLUS (GE, Cat # 17-1440-02), PBMC cells were isolated from EDTA anticoagulated whole blood (peripheral blood of 5 initial diagnosed HIV-positive patients from the key laboratory of STD/AIDS, You An Hospital, numbered 061302, 061903, 061904, 062003, 062004, respectively) according to the kit manual instruction. Specifically, the blood was centrifuged with a high-speed refrigeration centrifuge (Thermo Scientific Sorvall ST40R) using a horizontal rotor, at 800g for 30min, with the centrifugation speed of increase being set at 3, the speed of decrease being set at 0, and the temperature being set at 20°C. After centrifugation, PBMC cell layer was isolated gently and placed into a new centrifuge tube. The PBMC was washed with DPBS with a volume of 3-5 times the volume of the cell layer and then centrifuged for 10min at 20°C, 300g. After centrifugation, the supernatant was removed. Then, pre-cooled erythrocyte lysate (Tianjin Haoyang NH4CL2009) was added to the cell precipitate, and placed at 4°C for 2 minutes to lyse erythrocytes. Subsequently, pre-cooled DPBS was added to make the total volume to be 40-45ml. The cells were blown up and down to mix, and centrifuged at 300g and 4°C for 10 min to obtain PBMC cells.

### 2. Isolation and culture of CD4 + T cells

Human CD4 + T cell isolation kit (Antibody Cocktail; Biotech-beads) was used for CD4+T cells Isolation. Specifically, based on the counting number of above-mentioned PBMC cells, 40ul MACS Buffer was added to per 1 × 10⁷ cells (cells less than 1.5 × 10⁷, counted as 1 × 10⁷; cells more than 1.5× 10⁷, counted as 2 × 10⁷, and so on) to resuspend cells, and then 10u1 Antibody Cocktail was added, thoroughly mixed, and incubated at room temperature without light for 10min. Then 30ul (equal proportion) MACS Buffer and 20ul Biotech-beads were added, mixed well, and incubated at room temperature without light for 10min. At the same time, the LS sorting column was placed on the sorting rack, a filter was placed on the sorting column, and 2ml MACS Buffer was added to wash twice. 1-2ml MACS Buffer was added to the incubated cell suspension, mixed well. Then the mixture was added to the washed cell sorting column, and the outflow suspension was collected. After the cell suspension ran out, 2ml MACS Buffer was added to wash the column twice and the outflow suspension was also collected. After the collected cell suspension was fully mixed, 300ul of cell suspension was taken for counting. 1ml T cell complete medium (rHuIL 2200IU/ml, 1X GlutaMAX) was added per 2 × 10⁶ cells to resuspend the cells.

CD4 + T cells were activated by magnetic beads coated with anti-CD3 and anti-CD28 antibodies, according to the instruction of the Dynabeads Human T-Activator CD3/CD28 for T-Cell Expansion and Activation kit (Thermofisher, 11131D). Firstly, based on the counting number of CD4 + T cells, the cells and washed CD3/CD28 magnetic beads were mixed, with 25ul magnetic beads of the original concentration per 1 × 10⁶ cells. Then the mixture was supplemented with IL-2 (R&D, 202-IL-010) with a final concentration of 30IU/ml and human serum albumin or human AB serum with a final concentration of 3-5%. After fully mixed, the mixture was cultured at 37 °C for 48 hours. The mixture of cells and magnetic beads was placed into a 15ml centrifuge tube, oscillated at 2000rpm for 30s, and then placed on a magnetic frame for 1 minute. The supernatant was aspirated into a new centrifuge tube. The cells were resuspended in complete culture medium, and centrifuged 300g for 5 minutes. After centrifugation, the supernatant was discarded, the cell precipitate was resuspended with fresh complete medium, and then some of the cells were taken for counting and flow cytometry.

### 3. CD4 + T cell infection and HIV viral load Detection

Untreated 24-well plates was coated with Recombinant Human Fibronectin Fragment (RetroNectin ^{®}) at a concentration of 10 µ g/cm² and incubated at 37 °C for 1 hour. Each well of the coated 24-well plates was washed with DPBS twice, 500 µ 11% PBS-BSA was then added, and incubated for blocking at 37 °C for 1 hour. Then each well was washed with DPBS for 3 times, and 500 µ 1 complete culture medium was then added. The recombinant lentivirus SIV-G3L2, SIV-G2N or SIV-GFP was respectively added to each group with MOI=20, and incubated at 37 °C for 1 hour. After 1 hour, 250 µ 1 culture solution was discarded from each well. The cells obtained above were centrifuged at 2000rpm for 5 minutes, and the supernatant was discarded. Then the cells were resuspended in 500 µ 1 medium, inoculated to each well, and cultured at 37 °C, 5% CO2. The above infection steps were repeated on day 2 and day 3, respectively. The culture supernatant was collected on the 7th and 14th day of culture, and the HIV-1 viral load was detected by HIV-1 nucleic acid quantitative detection kit (QPCR) (Daan gene). According to the instruction of the kit, virus nucleic acid was extracted, and QPCR system was prepared, with the reaction procedure of 50 °C, 15min; 95 °C for 15min; (94 °C 15sec, 55 °C 45sec) × 45 cycles. Using FAM/TAMRA and VIC/NONE double fluorescence detection, the results of multiple batches of samples were listed in Table 12.

**Table 12 Inhibitory effects of SIV-G3L2 and SIV-G2N dual-targets drugs on HIV replication in CD4 + T cells of HIV patients**

| Patient number No. | Injected drugs | Viral load-day7(copies/µL) | Viral load-day14(copies/µL |
|---|---|---|---|
| 061903 | SIV-GFP | 2.8E+04 | - |
| | SIV-G3L2 | 3.1E+04 | - |
| | SIV-G2N | 3.4E+04 | - |
| 061904 | SIV-GFP | 3.9 E+04 | - |
| | SIV-G3L2 | 4.8E+02 | - |
| | SIV-G2N | 6.2E+02 | - |
| 061302 | SIV-GFP | 3.5E+06 | 3.7E+03 |
| | SIV-G3L2 | 9.1E+05 | 1.2E+02 |
| | SIV-G2N | 5.4E+05 | 2.1 E+02 |
| 062003 | SIV-GFP | 1.4E+05 | - |
| | SIV-G3L2 | 3.6E+04 | - |
| | SIV-G2N | 2.8E+04 | - |
| 062004 | SIV-GFP | 3.5 E+03 | - |
| | SIV-G3L2 | undected | - |
| | SIV-G2N | undected | - |

As shown in Table 12, recombinant lentivirus drugs SIV-G3L2 or SIV-G2N could effectively inhibit HIV virus replication in CD4 + T cells of HIV positive patients. After 7 days of in vitro culture, the viral load in CD4 + T cell culture supernatant from several patients decreased significantly.

The above are merely preferred embodiments of the present invention, and are not intended to limit the scope of protection of the present invention. Any modifications, equivalent replacements, improvements, etc. made within the spirit and principle of the present invention are all included within the scope of protection of the present invention.

## Claims

1. An RNA for targeting HIV, wherein the sequence is shown in any one of SEQ ID NOs: 6-10 or 20-24.

2. A siRNA targeting HIV, wherein the siRNA comprises a sense strand and a complementary antisense strand, and consists of the following sequences:
(i) the sequence of the sense strand shown in SEQ ID NO: 6, and the sequence of the antisense strand shown in SEQ ID NO: 20;
(ii) the sequence of the sense strand shown in SEQ ID NO: 7 and the sequence of the antisense strand shown in SEQ ID NO: 21;
(iii) the sequence of the sense strand shown in SEQ ID NO: 8 and the sequence of the antisense strand shown in SEQ ID NO: 22;
(iv) the sequence of the sense strand shown in SEQ ID NO: 9 and the sequence of the antisense strand shown in SEQ ID NO: 23; or
(v) the sequence of the sense strand shown in SEQ ID NO: 10, and the sequence of the antisense strand shown in SEQ ID NO: 24.

3. A shRNA targeting HIV, wherein the shRNA comprises any one of the sequences as claimed in claim 1 or 2.

4. The shRNA as claimed in claim 3, wherein the sequence of the shRNA is shown in any one of SEQ ID NOs: 25-29.

5. A nucleic acid coding for shRNA as claimed in claim 3 or 4.

6. The nucleic acid coding for shRNA as claimed in claim 5, the sequence of which is shown in any one of SEQ ID NOs: 11-15.

7. A combination of RNA targeting HIV, wherein the combination comprises at least one of the RNA of claim 1.

8. A combination of siRNAs targeting HIV, wherein the combination comprises at least one of the siRNAs of claim 2.

9. A combination of shRNAs targeting HIV, wherein the combination comprises at least one of the shRNAs of claim 3 or 4.

10. A combination of the nucleic acids coding for shRNAs targeting HIV, wherein the combination comprises at least one of the nucleic acids coding for the shRNAs of claim 5 or 6.

11. A combination of RNA targeting HIV, wherein the combination comprises at least two of the followings: the RNA targeting the Gag gene, the RNA targeting the LTR gene, or the RNA targeting the Nef gene.

12. The combination of the RNAs as claimed in claim 11, wherein the combination comprises:
(i) an RNA targeting the Gag gene and an RNA targeting the LTR gene,
(ii) an RNA targeting the Gag gene and an RNA targeting the Nef gene, or
(iii) an RNA targeting the LTR gene and an RNA targeting the Nef gene.

13. The combination of the RNAs as claimed in claim 11 or 12, wherein:
the sequence of RNA targeting the Gag gene of HIV is selected from the sequences consisting of 19-23 continuous nucleotides in any one of SEQ ID NOs: 1-3 or the sequences reversely complementary thereto, preferably, the sequence of RNA is shown in any one of SEQ ID NOs: 6-8 or 20-22.
the sequence of RNA targeting the LTR gene of HIV is selected from sequences consisting of 19-26 continuous nucleotides in SEQ ID NO: 4 or the sequences reversely complementary thereto, preferably, the sequence of RNA is shown in SEQ ID NO: 9 or 23;
the sequence of RNA targeting the Nef gene of HIV is selected from the sequences consisting of 19-25 continuous nucleotides in SEQ ID NO: 5 or the sequences reversely complementary thereto, preferably, the sequence of RNA is shown in SEQ ID NO: 10 or 24.

14. The combination of the RNAs as claimed in any one of claims 11-13, wherein the combination comprises:
(i) an RNA targeting the Gag gene and an RNA targeting the LTR gene, wherein the sequence of RNA targeting the Gag gene is shown in SEQ ID NO: 6 or 20, and the sequence of RNA targeting the LTR gene is shown in SEQ ID NO: 9 or 23;
(ii) an RNA targeting the Gag gene and an RNA targeting the LTR gene, wherein the sequence of RNA targeting the Gag gene is shown in SEQ ID NO: 7 or 21, and the sequence of RNA targeting the LTR gene is shown in SEQ ID NO: 9 or 23;
(iii) an RNA targeting the Gag gene and an RNA targeting the LTR gene, wherein the sequence of RNA targeting the Gag gene is shown in SEQ ID NO: 8 or 22, and the sequence of RNA targeting the LTR gene is shown in SEQ ID NO: 9 or 23; or
(iv) an RNA targeting the Gag gene and an RNA targeting the Nef gene, wherein the sequence of RNA targeting the Gag gene is shown in SEQ ID NO: 7 or 21, and the sequence of RNA targeting the Nef gene is shown in SEQ ID NO: 10 or 24.

15. A combination of siRNAs targeting HIV, wherein the combination comprises at least two of the followings:
the siRNA targeting the Gag gene, the siRNA targeting the LTR gene, or the siRNA targeting the Nef gene.

16. The combination of siRNAs as claimed in claim 15, wherein, the combination comprises:
(i) a siRNA targeting the Gag gene and a siRNA targeting the LTR gene,
(ii) a siRNA targeting the Gag gene and a siRNA targeting the Nef gene, or
(iii) a siRNA targeting the LTR gene and a siRNA targeting the Nef gene.

17. The combination of siRNAs as claimed in claim 15 or 16, wherein:
the sense strand sequence of the siRNA targeting the Gag gene of HIV is selected from the sequences consisting of 19-23 continuous nucleotides in any one of SEQ ID NOs: 1-3, preferably is shown in any one of SEQ ID NOs: 6-8, and the antisense strand sequence is a sequence which is reversely complementary to the sense strand;
the sense strand sequence of the siRNA targeting the LTR gene of HIV is selected from the sequences consisting of 19-26 continuous nucleotides in SEQ ID NO: 4, preferably is shown in SEQ ID NO: 9, and the antisense strand sequence is a sequence which is reversely complementary to the sense strand;
the sense strand sequence of the siRNA targeting the Nef gene of HIV is selected from the sequence consisting of 19-25 continuous nucleotides in SEQ ID NO: 5, preferably is shown in SEQ ID NO: 10, and the antisense strand sequence is a sequence which is reversely complementary to the sense strand.

18. The combination of siRNAs as claimed in any one of claims 15-17, wherein the combination comprises:
(i) a siRNA targeting the Gag gene and a siRNA targeting the LTR gene, wherein the sense strand sequence of the siRNA targeting the Gag gene is shown in SEQ ID NO: 6, the antisense strand sequence is shown in SEQ ID NO: 20, and the sense strand sequence of the siRNA targeting the LTR gene is shown in SEQ ID NO: 9, the antisense strand sequence is shown in SEQ ID NO: 23;
(ii) a siRNA targeting the Gag gene and a siRNA targeting the LTR gene, wherein the sense strand sequence of the siRNA targeting the Gag gene is shown in SEQ ID NO: 7, the antisense strand sequence is shown in SEQ ID NO: 21, and the sense strand sequence of the siRNA targeting the LTR gene is shown in SEQ ID NO: 9, the antisense strand sequence is shown in SEQ ID NO: 23;
(iii) a siRNA targeting the Gag gene and a siRNA targeting the LTR gene, wherein the sense strand sequence of the siRNA targeting the Gag gene is shown in SEQ ID NO: 8, the antisense strand sequence is shown in SEQ ID NO: 22, and the sense strand sequence of the siRNA targeting the LTR gene is shown in SEQ ID NO: 9, the antisense strand sequence is shown in SEQ ID NO: 23; or
(iv) a siRNA targeting the Gag gene and a siRNA targeting the Nef gene, wherein the sense strand sequence of the siRNA targeting the Gag gene is shown in SEQ ID NO: 7, the antisense strand sequence is shown in SEQ ID NO: 21, and the sense strand sequence of the siRNA targeting the Nef gene is shown in SEQ ID NO: 10, the antisense strand sequence is shown in SEQ ID NO: 24.

19. A combination of shRNAs targeting HIV, wherein the combination comprises at least two of the followings: the shRNA targeting the Gag gene, the shRNA targeting the LTR gene, or the shRNA targeting the Nef gene.

20. The combination of shRNAs targeting HIV as claimed in claim 19, wherein the combination comprises:
(i) a shRNA targeting the Gag gene and a shRNA targeting the LTR gene,
(ii) a shRNA targeting the Gag gene and a shRNA targeting the Nef gene, or
(iii) a shRNA targeting the LTR gene and a shRNA targeting the Nef gene.

21. The combination of shRNAs targeting HIV as claimed in claim 19 or 20, wherein:
the sequence of shRNA targeting the Gag gene comprises: (i) the sequences shown in SEQ ID NO: 6 and SEQ ID NO: 20; (ii) the sequences shown in SEQ ID NO: 7 and SEQ ID NO: 21; or (iii) the sequences shown in SEQ ID NO: 8 and SEQ ID NO: 22;
the sequence of shRNA targeting the LTR gene comprises the sequences shown in SEQ ID NO: 9 and SEQ ID NO: 23;
the sequence of shRNA targeting the Nef gene comprises the sequences shown in SEQ ID NO: 10 and SEQ ID NO: 24.

22. The combination of shRNAs targeting HIV as claimed in any one of claims 19-21, wherein the combination comprises:
(i) a shRNA targeting the Gag gene and a shRNA targeting the LTR gene, wherein the sequence of the shRNA targeting the Gag gene comprises the sequences shown in SEQ ID NO: 6 and SEQ ID NO: 20, and the sequence of the shRNA targeting the LTR gene comprises the sequences shown in SEQ ID NO: 9 and SEQ ID NO: 23;
(ii) a shRNA targeting the Gag gene and a shRNA targeting the LTR gene, wherein the sequence of the shRNA targeting the Gag gene comprises the sequences shown in SEQ ID NO: 7 and SEQ ID NO: 21, and the sequence of the shRNA targeting the LTR gene comprises the sequences shown in SEQ ID NO: 9 and SEQ ID NO: 23;
(iii) a shRNA targeting the Gag gene and a shRNA targeting the LTR gene, wherein the sequence of shRNA targeting the Gag gene comprises the sequences shown in SEQ ID NO: 8 and SEQ ID NO: 22; the sequence of shRNA targeting the LTR gene comprises sequences shown in SEQ ID NO: 9 and SEQ ID NO: 23; or
(iv) a shRNA targeting the Gag gene and a shRNA targeting the Nef gene, wherein the sequence of the shRNA targeting the Gag gene comprises the sequences shown in SEQ ID NO: 7 and SEQ ID NO: 21, and the sequence of the shRNA targeting the Nef gene comprises the sequences shown in SEQ ID NO: 10 and SEQ ID NO: 24.

23. The combination of shRNAs targeting HIV as claimed in claim 19 or 20, wherein:
the shRNA targeting the Gag gene is selected from the sequences shown in any one of SEQ ID NOs: 25 -27;
the shRNA targeting the LTR gene is the sequence shown in SEQ ID NO: 28;
the shRNA targeting the Nef gene is the sequence shown in SEQ ID NO: 29.

24. The combination of shRNAs targeting HIV as claimed in any one of claims 19-23, wherein the combination comprises:
(i) a shRNA targeting the Gag gene and a shRNA targeting the LTR gene, wherein the sequence of the shRNA targeting the Gag gene is shown in SEQ ID NO: 25, and the sequence of the shRNA targeting the LTR gene is shown in SEQ ID NO: 28;
(ii) a shRNA targeting the Gag gene and a shRNA targeting the LTR gene, wherein the sequence of the shRNA targeting the Gag gene is shown in SEQ ID NO: 26, and the sequence of the shRNA targeting the LTR gene is shown in SEQ ID NO: 28;
(iii) a shRNA targeting the Gag gene and a shRNA targeting the LTR gene, wherein the sequence of the shRNA targeting the Gag gene is shown in SEQ ID NO: 27; the sequence of the shRNA targeting the LTR gene is shown in SEQ ID NO: 28; or
(iv) a shRNA targeting the Gag gene and a shRNA targeting the Nef gene, wherein the sequence of the shRNA targeting the Gag gene is shown in SEQ ID NO: 26; and the sequence of the shRNA targeting the Nef gene is as shown in SEQ ID NO: 29.

25. A combination of the nucleic acids coding for shRNAs targeting HIV, wherein the combination comprises at least two of the followings: the nucleic acid coding for shRNA targeting the Gag gene, the nucleic acid coding for shRNA targeting the LTR gene, or the nucleic acid coding for shRNA targeting the Nef gene.

26. The combination of the nucleic acids coding for shRNAs targeting HIV as claimed in claim 25, wherein the combination comprises:
(i) a nucleic acid coding for shRNA targeting the Gag gene and a nucleic acid coding for shRNA targeting the LTR gene,
(ii) a nucleic acid coding for shRNA targeting the Gag gene and a nucleic acid coding for shRNA targeting the Nef gene, or
(iii) a nucleic acid coding for shRNA targeting the LTR gene and a nucleic acid coding for shRNA targeting the Nef gene.

27. The combination of the nucleic acids coding for shRNAs targeting HIV as claimed in claim 25 or 26, wherein:
the shRNA targeting the Gag gene comprises: (i) the sequence shown in SEQ ID NO: 6 and SEQ ID NO: 20; (ii) the sequence shown in SEQ ID NO: 7 and SEQ ID NO: 21; or (iii) the sequence shown in SEQ ID NO: 8 and SEQ ID NO: 22;
the shRNA targeting the LTR gene comprises the sequence shown in SEQ ID NO: 9 and SEQ ID NO: 23;
the shRNA targeting the Nef gene comprises the sequences shown in SEQ ID NO: 10 and SEQ ID NO: 24.

28. The combination of the nucleic acids coding for shRNAs targeting HIV as claimed in any one of claims 25-27, wherein the combination comprises:
(i) a nucleic acid coding for shRNA targeting the Gag gene and a nucleic acid coding for shRNA targeting the LTR gene, wherein the shRNA targeting the Gag gene comprises the sequences shown in SEQ ID NO: 6 and SEQ ID NO: 20, and the shRNA targeting the LTR gene comprises the sequences shown in SEQ ID NO: 9 and SEQ ID NO: 23;
(ii) a nucleic acid coding for shRNA targeting the Gag gene and a nucleic acid coding for shRNA targeting the LTR gene, wherein the shRNA targeting the Gag gene comprises the sequences shown in SEQ ID NO: 7 and SEQ ID NO: 21, and the shRNA targeting the LTR gene comprises the sequences shown in SEQ ID NO: 9 and SEQ ID NO: 23;
(iii) a nucleic acid coding for shRNA targeting the Gag gene and a nucleic acid coding for shRNA targeting the LTR gene, wherein the shRNA targeting the Gag gene comprises the sequence shown in SEQ ID NO: 8 and SEQ ID NO: 22; shRNA targeting the LTR gene comprises the sequence shown in SEQ ID NO: 9 and SEQ ID NO: 23; or
(iv) a nucleic acid coding for shRNA targeting the Gag gene and a nucleic acid coding for shRNA targeting the Nef gene, wherein the shRNA targeting the Gag gene comprises the sequences shown in SEQ ID NO: 7 and SEQ ID NO: 21, and the shRNA targeting the Nef gene comprises the sequences shown in SEQ ID NO: 10 and SEQ ID NO: 24.

29. The combination of the nucleic acids coding for shRNAs targeting HIV as claimed in claim 25 or 26, wherein:
the sequence of the nucleic acid coding for the shRNA targeting the Gag gene is selected from the sequences shown in any one of SEQ ID NOs: 11 -13.
the sequence of the nucleic acid coding for the shRNA targeting the LTR gene is the sequence shown in SEQ ID NO: 14.
the sequence of the nucleic acid coding for the shRNA targeting the Nef gene is the sequence shown in SEQ ID NO: 15.

30. The combination of the nucleic acids coding for shRNAs targeting HIV as claimed in any one of claims 25-29, wherein the combination comprising:
(i) a nucleic acid coding for shRNA targeting the Gag gene and a nucleic acid coding for shRNA targeting the LTR gene, wherein the sequence of the nucleic acid coding for the shRNA targeting the Gag gene is shown in SEQ ID NO: 11, and the sequence of the nucleic acid coding for the shRNA targeting the LTR gene is shown in SEQ ID NO: 14,
(ii) a nucleic acid coding for shRNA targeting the Gag gene and a nucleic acid coding for shRNA targeting the LTR gene, wherein the sequence of the nucleic acid coding for the shRNA targeting the Gag gene is shown in SEQ ID NO: 12, and the sequence of the nucleic acid coding for the shRNA targeting the LTR gene is shown in SEQ ID NO: 14,
(iii) a nucleic acid coding for shRNA targeting the Gag gene and a nucleic acid coding for shRNA targeting the LTR gene, wherein the sequence of the nucleic acid coding for the shRNA targeting the Gag gene is shown in SEQ ID NO: 13, and the sequence of the nucleic acid coding for the shRNA targeting the LTR gene is shown in SEQ ID NO: 14, or
(iv) a nucleic acid coding for shRNA targeting the Gag gene and a nucleic acid coding for shRNA targeting the Nef gene, wherein the sequence of the nucleic acid coding for the shRNA targeting the Gag gene is shown in SEQ ID NO: 12, and the sequence of the nucleic acid coding for the shRNA targeting the Nef gene is shown in SEQ ID NO: 15.

31. An expression cassette comprising the nucleic acid coding for shRNA as claimed in any one of claims 5, 6 or the combination of the nucleic acids coding for shRNAs as claimed in any one of claims 10, or 25-30.

32. The expression cassette as claimed in claim 31, which further comprises a promoter, wherein the promoter is RNA polymerase II promoter or RNA polymerase III promoter; preferably RNA polymerase III promoter, said RNA polymerase II promoter or RNA polymerase III promoter further comprises a synthetic or modified DNA fragment.

33. The expression cassette as claimed in claim 32, wherein the RNA polymerase III promoter is selected from H1 promoter, U6 promoter, or 7SK promoter.

34. The expression cassette as claimed in any one of claims 31-33, wherein the expression of shRNA targeting the Gag gene is driven by H1 promoter and the expression of shRNA targeting LTR or Nef is driven by U6 promoter, or the expression of shRNA targeting the Gag gene is driven by U6 promoter, and the expression of shRNA targeting LTR or Nef is driven by H1 promoter.

35. A vector comprising the RNA of claim 1, the siRNA of claim 2, the shRNA of claim 3 or 4, the nucleic acid coding for shRNA of claim 5 or 6, the combination of RNAs of any one of claims 7 or 11-14, the combination of siRNAs of any one of claims 8 or 15-18, the combinations of shRNAs of any one of claims 9 or 19-24, or the combination of the nucleic acids coding for shRNAs of any one of claims 10 or 25-30, or the expression cassette of any one of claims 31-34.

36. The vector as claimed in claim 35, wherein the vector comprises two or more expression cassettes, wherein the two or more expression cassettes are contained in the same vector, or the two or more expression cassettes are contained in different vectors, respectively.

37. The vector as claimed in claim 35 or 36, wherein the vector is selected from the group consisting of plasmid vector, viral vector, liposome, nanoparticle, or exosome; wherein the viral vector is selected from adeno-associated viral vector, lentiviral vector or adenoviral vector.

38. The vector as claimed in claim 37, wherein the lentiviral vector is selected from the group consisting of human immunodeficiency virus (HIV-1 and HIV-2) vector, simian immunodeficiency virus (SIV) vector, feline immunodeficiency virus (FIV) vector, equine infectious anemia virus (EIAV) vector and goat arthritis encephalitis virus (CAEV) vector.

39. A cell, wherein the cell comprises the RNA of claim 1, the siRNA of claim 2, The shRNA of claim 3 or 4, the nucleic acid coding for shRNA of claim 5 or 6, the combination of RNAs of any one of claims 7 or 11-14, the combination of siRNAs of any one of claims 8 or 15-18, the combination of shRNAs of any one of claims 9 or 19-24, the combination of the nucleic acids coding for shRNAs of any one of claims 10 or 25-30, the expression cassette of any one of claims 31-34, or the vector of any one of claims 35-38.

40. The cell as claimed in claim 39, wherein the cell is derived from HIV receptor cell, peripheral blood mononuclear cell or lymphocyte; preferably, the cell are derived from T cell (eg, naive T cell, memory T cell, effector T cell, cytotoxic T cell, helper T cell, regulatory T cell, CD4 + T cell, CD8 + T cell, NKT cell, γ δ T cell), NK cell, antigen presenting cell (e.g. macrophage, dendritic cell).

41. The cell as claimed in claim 39, wherein the cell is derived from stem cell including, but not limited to, hematopoietic stem cell, hematopoietic progenitor cell, memory T stem cell (e.g. central memory T cell, effector memory T cell, or stem cell-like memory T cell).

42. The cell as claimed in any one of claims 39-41, wherein the cell is derived from mammal, primate, or human.

43. The cell as claimed in claim 39, wherein the cell is derived from engineered cell.

44. A pharmaceutical composition comprising the RNA of claim 1, the siRNA of claim 2, the shRNA of claim 3 or 4, the nucleic acid coding for shRNA of claim 5 or 6, the combination of the RNAs of any one of claims 7 or 11-14, the combination of siRNAs of any one of claims 8 or 15-18, the combination of shRNAs of any one of claims 9 or 19-24, the combination of the nucleic acids coding for shRNAs of any one of claims 10 or 25-30, the expression cassette of any one of claims 31-34, or the vector of any one of claims 35-38, or the cell of any one of claims 39-43, and a pharmaceutically acceptable carrier.

45. The pharmaceutical composition as claimed in claim 44, wherein the pharmaceutical composition further comprises other anti-HIV drugs including, but not limited to, nucleoside inhibitors, HIV vaccines, broad-spectrum neutralizing antibodies, CAR-T cells.

46. The use of the RNA of claim 1, the siRNA of claim 2, the shRNA of claim 3 or 4, the nucleic acid coding for shRNA of claim 5 or 6, the combination of the RNAs of any one of claims 7 or 11-14, the combination of siRNAs of any one of claims 8 or 15-18, the combination of shRNAs of any one of claims 9 or 19-24, the combination of the nucleic acids coding for shRNAs of any one of claims 10 or 25-30, the expression cassette of any one of claims 31-34, or the vector of any one of claims 35-38, or the cell of any one of claims 39-43, or the pharmaceutical composition of claims 44 or 45 for the preparation of a drug for treating HIV infection or AIDS.

47. A method for preventing and/or treating HIV infection or AIDS, comprising administering to a patient the RNA of claim 1, the siRNA of claim 2, the shRNA of claim 3 or 4, the nucleic acid coding for shRNA of claim 5 or 6, the combination of the RNAs of any one of claims 7 or 11-14, the combination of siRNAs of any one of claims 8 or 15-18, the combination of shRNAs of any one of claims 9 or 19-24, the combination of the nucleic acids coding for shRNAs of any one of claims 10 or 25-30, the expression cassette of any one of claims 31-34, or the vector of any one of claims 35-38, or the cell of any one of claims 39-43, or the pharmaceutical composition of claim 44 or 45.

48. The method as claimed in claim 47, further comprising administering to the patient other anti-HIV drugs including, but not limited to, nucleoside inhibitors, HIV vaccines, broad-spectrum neutralizing antibodies, CAR-T cells.
